# EUROPEAN PATENT APPLICATION

(11) **EP 1 329 827 A1**
(43) Date of publication of application: **23.07.2003**
(21) Application number: 01976701.1
(22) Date of filing: 17.10.2001
(51) Int. Cl.: G06F 17/60

(54) **INPUT ASSISTING SYSTEM**

(30) Priority: 18.10.2000 JP 2000317608
(71) Applicant: ARKRAY, Inc., Kyoto-shi, Kyoto 601-8045 (JP)
(72) Inventor: OOTSUKA, Kazushi, c/o ARKRAY, INC., Kyoto-shi, Kyoto 601-8045 (JP)
(74) Representative: Piésold, Alexander J.
(86) International application number: JP0109092
(87) International publication number: WO02033614

(57) **Abstract**

An input assisting system in which information on past defective data that are causes of return is shared and input data is checked with reference to the information. Computers (1) of medical institutions (A,B,C) are connected to a server system (2) through the Internet (3). Return information concerning past return cases collected from the institutions (A,B,C) is stored in an error adjustment DB server (23) of the server system (2). An application server (21) of the server system (2) creates a receipt of a predetermined create on the basis of the data inputted from the computers (1) and checks and corrects the receipts with reference to the return information in the error adjustment DB server (23).

## Description

### Technical Field

The present invention relates to an input support system for checking the correctness of data inputted in a computer and suggesting revision of defective data.

### Background Art

Conventionally input support systems that check the correctness of inputted data in a simple manner at the time of input work by human beings into computers, and suggest revision of defective data are known. The input support systems are used widely in various fields. For example, in medical systems, receipt creation support systems or the like used in receipt creation institutions (medical institutions or the like) performing receipt creation operations are known. Though the above-mentioned medical institutions represent the receipt creation institutions, ethical pharmacies handling prescriptions issued at medical institutions or the like also are included in the receipt creation institutions. Though a conventional receipt creation support system will be described below for an explanation of background art, the applications of the present invention will not be limited to the receipt creation support system.

The following description concerns the handling of receipts in medical institutions.

A medical institution receives medical service fees for medical practice to a patient. The medical service fees include a fixed share from a medical insurance and a fixed share from a patient (the insured) under the health insurance law. A medical insurance system is established by insureds who pay the premiums and collect on the insurance, and insurers who are financed by collecting the premiums and allocate the funds when the insureds are insured.

A medical institution charges a patient directly for the part of the medical service fees for the part that an insured should share. Regarding the share of the medical insurance in the medical service fees, the medical institution charges the insurer through an independent institution. This independent institution is called the Fund (Social Insurance Medical Fee Payment Fund) for the Social Insurance, or the Federation (All-Japan Federation of National Health Insurance Organization) for the National Health Insurance.

A document submitted from a medical institution to the independent institution for the purpose of charging medical service fees is called a medical service fee specification (receipt). The independent institution performs payment of the medical service fee to the medical institution after checking the receipt submitted from the medical institution for clerical errors, e.g., errors in the calculation of insurance scores.

A receipt is created as a document for each patient by compiling medical service fees of one month (closed at month-end, and submitted by the 9^{th} or 10^{th} next month). The created receipts are divided into ones directed to the Fund and ones to the Federation, and summed up in a general list.

A conventional receipt creation support system is a system for supporting only creation of a receipt for each patient and the general list in a medical institution. A receipt submitted from a medical institution is audited at the Fund or the Federation, and when there are defects or errors in the description, the receipt will be returned to the medical institution. This is called a direct return. In a case of a return, the medical institution must correct the returned receipt and re-submit for a second audit.

Similarly, when any payment faults caused by defects in the receipt are found as a result of checking and confirmation by the insurer (the Social Insurance or the National health Insurance) after the receipt is once accepted and a medical service fee is paid from the Fund or the Federation, the submitted receipt is returned through the Fund or the Federation to the medical institution. This is called an indirect return. Similarly in this case, the receipt should be corrected and re-submitted.

In general, the direct return is carried out by the end of the month in which the receipt is submitted, and the indirect return is carried out at around the month-end after two months from the submission of the receipt. As described above, when the receipt is returned due to defects, the receipt should be submitted again, and this will increase paperwork in medical services. Another problem is that, for a case of a direct return, payment of the medical service fee for the receipt will be delayed.

Another well-known example of a conventional input support system is a managerial decision request system used in corporations or firms. A managerial decision request system denotes a system for supporting creation of a managerial decision request to obtain settlement required for conducting business in the firm. However, the conventional managerial decision request system aims to prevent congestion in the circulation of documents, while it has no function to check defects in the description. Therefore, it cannot prevent settlement delay caused by a return of the managerial decision request.

As mentioned above, not limiting to the receipt creation support system or the in-house managerial decision request system, conventional input support systems include an object to be achieved, i.e., revising defects in input data more appropriately.

For achieving the object, the present invention provides an input support system that can check defects in input data by using past examples and revise defects in the input data more appropriately.

### Disclosure of Invention

For achieving the above-mentioned objects, an input support system according to the present invention includes a client system for performing data input work, and a server system that is connected to one or plural client system(s) and uses defective data information collected from each client system so as to support the data input work in the client system, wherein a defective data information storing portion for storing the defective data information as a record including data to be revised and revised data as items is provided in at least one of the server system and the client system, and a check processing portion for performing a process of checking input data is provided in either the client system or the server system, the check process denotes a process of contrasting the defective data information in the defective data information storing portion and the input data in the client system, and when there are input data the same as the data included in an item of data to be revised in the defective data information, performing a defect prevention process or a defect revision process.

According to this system, the check processing portion contrasts input data with past defective data information stored in the defective data information storing portion so as to check whether there are any input data identical to description data that have caused remand. When there are such defective data, the check processing portion performs a defect prevention process or a defect revision process with respect to the description data.

The defect prevention process denotes a process for preventing erroneous inputting of data that may lead to defects in a relationship with the targeted description data, and it includes processes, for example, a process of suggesting correct description data. The defect revision process denotes a process of revising the data to be processed to a piece of correct data.

The input support system provided in this manner can check and revise input data by using the past defective data information in order to prevent at least remand caused by a reason that already has occurred.

As described above, in the input support system, the defective data information storing portion is provided either in the server system or the client system, or both. When the defective data information storing portion is provided in the client system, for example, the server system can acquire defective data information from a defective data information storing portion(s) of one or plural client system(s), perform a process of checking the input data at the check processing portion within the server system, and send the result to the same or other client system. Alternatively, the server system can acquire defective data from a defective data information storing portion(s) of one or plural client system(s), and send the data to another client system in order to perform a process of checking the input data in this client system.

In the input support system, it is preferable that the defect prevention process by the check processing portion includes a process of extracting defective data information relating to the data to be processed from the defective data information storing portion and presenting it.

According to the configuration, since past examples with respect to the same data as the inputted data are presented, repetition of similar errors will be prevented.

In the input support system, it is preferable that the client system includes a defective data information transmitting portion for transmitting the defective data information relating to the input data of the client system to the server system, and the server system stores remand information sent from the client system, in the defective data information storing portion.

According to the configuration, defective data information can be collected efficiently, and the defective data can be checked more precisely and revised exactly. Preferably, the remand information is return information in the receipt.

For achieving the above-mentioned object, an input support system of the present invention includes a client system provided in a receipt creation institution for performing a receipt creation business, and a server system that is connected from a client system(s) of one or plural receipt creation institution(s) through a communication line and that supports the receipt creation business at the client system(s) by using the receipt return information collected from each receipt creation institution, where a return information storing portion that stores the return information as a record including data to be returned and revised data as items is provided in the server system, a check processing portion for performing a process of checking the description data of the receipt before submission is provided in either the client system or the server system, the check process denotes a process of contrasting the return information of the return information storing portion with description data of the receipt before submission, and when there exist description data that are the same as the data included in the item of data to be returned in the return information, performing a defect prevention process or a defect revision process with respect to the description data as the data to be processed.

According to the configuration, the check processing portion contrasts the past return information stored in the return information storing portion with the description data of the receipt before being submitted to the audit institution, so that it can check the receipt to be submitted as to whether there are any data identical to description data that have caused a return of the receipt. If there are such description data, the check processing portion performs a defect prevention process or a defect revision process which regards the description data as data to be processed.

The defect prevention process denotes a process for preventing erroneous inputting of data that may cause the defect in the relationship with the description data as data to be processed, and it includes, for example, a process of suggesting correct description data. The defect revision process denotes a process of revising the data to be processed to correct data when the description data is defective.

As described above, by using the past receipt return information so as to check and revise the description of the receipt to be submitted for the purpose of preventing a return caused by at least the same reason as the past one, an input support system for supporting a receipt creation business in a receipt creation institution can be provided.

Since the return information storing portion should be shared by client systems, it is provided in the server system. On the other hand, the check processing portion can have a configuration to be arranged in each client system so as to check only the receipt of the receipt creation institution, or it can have a configuration to be arranged in the server system so as to process the receipts of all the client systems together.

In the input support system, it is preferable that the process of preventing data defect by the check processing portion includes a process of extracting return information relating to the data to be processed from the return information storing portion and presenting it.

According to the configuration, since past examples with respect to the data that are the same as the inputted data (data to be processed) are presented by the return information in the case of, for example, a process of checking the inputted data at the same time of data input work, repetition of similar errors will be prevented, and thus a receipt free from a return can be created.

In the input support system, it is preferable that the process of revising the defect by the check processing portion includes a process of replacing the data to be processed by revised data of the return information including, in its item of data to be returned, the data that are the same as the data to be processed.

According to this configuration, in a case of performing erroneous input same as an example of a past return, the erroneously inputted part is replaced by revised data registered in the return information so as to revise correctly a receipt having a defect before submission, enabling the creation of a receipt that will not be returned.

In the input support system, it is preferable that the client system includes a return information transmitting portion for transmitting to the server system through the communications line the return information with respect to the receipt of the receipt creation institution, and the server system includes a return information registering portion for receiving the return information transmitted from the client system and allowing the return information storing portion to store the return information.

According to this configuration, the server system receives the return information from the client systems through the communications line, so that the return information can be collected efficiently.

In the input support system, it is preferable that the record of each return information at the return information storing portion further includes an item representing uneven distribution of the return information, and the check processing portion contrasts a district where a receipt creation institution that creates the receipt exists and the item of the uneven distribution for each return information so as to select return information to be referred to at the time of the check process.

The Fund or the Federation as the receipt audit institution has offices in respective prefectures, and it performs its receipt audit for every prefecture. For this reason, audit standards may vary for the details among the prefectures. According to the configuration, the receipt check process in every receipt creation institution can be performed on the basis of the return information corresponding to the district where the receipt creation institutions exist. Thereby, a check can be performed suitably for the audit standards in each district, and accuracy in the process of receipt check is improved.

In the input support system, it is preferable that the server system further comprises an uneven-distribution determining portion for determining the regional uneven distribution of the return information having identical contents in the return information storing portion and registering the result in the regionality item of the return information.

According to this configuration, the uneven-distribution determining portion determines the regional uneven distribution of the return information collected from the nationwide receipt creation institutions, and the result is registered in the regionality item for each return information so as to allow objective determination with respect to the regional uneven distribution among the districts.

In the input support system, it is preferable that a regional unit of the regional uneven distribution is a unit administered by an audit institution.

According to this configuration, the regional uneven distribution of the return information is determined on the basis of a unit administered by each audit institution, and thus a check process about each receipt can be performed by selectively using examples of the past return in the audit institution that will check the receipt. This serves to perform a check process that is accurate and adapted to the audit standards of the respective districts.

In the input support system, it is preferable that the attribute of the item for the regional uneven distribution of the return information records is selected from local information, region information, and national common information.

Prefecture information denotes return information unevenly distributed in particular prefectures. Since the receipt audit institutions are established on a prefecture basis, classification of the return information on the basis of the prefectures will enable to perform a highly accurate check process that is most suitable for the audit standards of the districts. Region information denotes information unevenly distributed to regions including plural prefectures, such as the Kanto Region. Since trends of diseases may differ among the regions due to the difference in the lifestyle habits and the climates, the classification of the return information into regions achieves an efficient check process. The national common information denotes return information free of a regional uneven distribution.

In the input support system, it is preferable that the client system includes a selecting portion for transmitting to the server system a use flag representing whether to use in the receipt creation institution each return information stored in the return information storing portion, where a record of each return information at the return information storing portion further includes a use flag item representing a use flag transmitted from the selecting portion of each receipt creation institution, and the check processing portion refers to the use flag item and selects return information to be referred to at the time of the check process.

According to this configuration, each receipt creation institution can select whether it uses every return information stored in the return information storing portion. Namely, each receipt creation institution designates any return information that is useless for the receipt creation institution as "no-use" so that the return information will not be displayed afterwards. This enables useful return information exclusively to each receipt creation institution to be provided so as to improve efficiency of the receipt creation business at the client system of the receipt creation institution.

In the input support system, it is preferable that the input support system further includes an input screen presenting portion for presenting an input screen that supports input of receipt description data, and a creating portion for creating a receipt on the basis of data inputted into the input screen.

According to this configuration, a process of creating the receipt as well as checking the receipt can be performed at the input support system. Since the check processing portion performs the check process with respect to the receipt created at the creating portion, the efficiency of the receipt creation work at each receipt creation institution can be improved.

In the input support system, it is preferable that the input screen presenting portion and the creating portion are provided in the client system.

According to this configuration, when inputting data into an input screen presented by the input screen presenting portion in the client system of the receipt creation institution, the creating portion creates a receipt based on the data. The thus created receipt is sent to a check processing portion provided in either the client system or the server system and subjected to a check process. Thereby, since each client system bears the process of receipt creation, the burden of the server system can be reduced as well in comparison with a configuration in which a server system performs creation of receipts.

In the input support system, it is preferable that the input screen presenting portion is provided in the client system and the creating portion is provided in the server system, and description data of the receipt inputted in the input screen is transmitted through the communications line to the creating portion of the server system.

According to this configuration, functions as a data inputting terminal will be sufficient for the client system, and the inputted data are sent to the creating portion of the server system and processed. Thereby, the configuration of the client system can be simplified. Moreover, since management of software or the like for realizing the creating portion can be carried out by the server system alone, the burden of management will be reduced.

In the input support system, it is preferable that the input screen presenting portion is provided in the server system and the creating portion is provided in the client system, where the input screen presenting portion transmits input screen data described in a page-description language to the client system through the communications line, the client system includes a display processing portion for displaying the input screen by analyzing the input screen data, and the display processing portion sends to the creating portion the description data of the receipt inputted into the input screen.

According to this configuration, since the inputted screen can be provided from the server system to the client system by means of a page-description language such as HTML, it is advantageous that, in a case of any modification or the like of the input screen, the modification can be reflected promptly. Furthermore, since the creating portion is provided in the client system, the server system will not be responsible for the receipt creation.

In the input support system, it is preferable that the input screen presenting portion and the creating portion are provided in the server system, the input screen presenting portion transmits the input screen data described in a page-description language to the client system through the communications line, the client system includes a display processing portion for displaying an input screen by analyzing the input screen data, and the display processing portion sends receipt description data inputted into the input screen to the creating portion of the server system through the communications line.

According to this configuration, since it is possible to present an input screen from the server system to the client system by means of a page-description language such as HTML, there is an advantage that, e.g., in the case of a modification in the input screen, the modification can be reflected promptly. Furthermore, since the creating portion is also provided in the server system, management of software or the like for realizing the creating portion can be carried out at the server system alone, thereby reducing the burden for management.

In the input support system, it is preferable that the input screen presenting portion acquires description data of a submitted receipt and displays on the input screen; the creating portion creates a receipt for resubmission on the basis of the description data revised on the input screen; and the creating portion includes a return information creating portion for creating a new return information record to be registered in the return information storing portion, regarding the description data to be revised as the item of data to be returned and also regarding the revised data as an item of revised data.

When there is a return from the audit institution with respect to the submitted receipt, each audit institution revises this receipt and creates a receipt for resubmission. At this time, according to the above-described configuration, the return information creating portion creates return information automatically on the basis of the description data to be revised and also the revised description data. Accordingly, it is possible to collect return cases of each receipt creation institution thoroughly without any manual operations. Moreover, this provides a highly accurate check process based on abundant return information.

In the input support system, it is preferable that every time the description data of a receipt are inputted into the input screen, the check processing portion performs the check process with respect to the description data.

According to this configuration, the process of checking the inputted data is performed in substantial real-time, thereby preventing erroneous input efficiently.

In the input support system, it is preferable that the client system or the server system includes further a reading portion for reading the created receipt from a storage medium in which the created receipt is stored, and that the check processing portion performs the check process in a batch with respect to whole description data of one or plural receipt(s) that are read out from the storage medium by the reading portion.

According to this configuration, the overhead required for delivery of the description data and the result of the check process among the relating blocks can be reduced in comparison with a configuration for performing a check process for each subject of the description data and sending the result back, and thus the time for the entire process may be shortened.

In the input support system, it is preferable that a result of the check process performed in a batch by the check processing portion is compiled in a list and presented to each receipt creation institution.

In the input support system, it is preferable that the server system includes a charge reduction calculating portion for deciding the reduction of a use fee to be imposed on every receipt creation institution, corresponding to at least the number of subjects or frequency that the return information is provided from every client system to the server system.

According to the configuration, the system use fee paid from each receipt creation institution to the manager of the server system will be reduced in accordance with at least either the number of the subjects or the frequency of the return information provided from the receipt creation institution to the server system. This mechanism can be an effective incentive for encouraging the receipt creation institution to positively provide the return information, especially when the return information is provided voluntarily from the respective receipt creation institutions.

In the input support system, it is preferable that the server system includes a return information use fee calculating portion for deciding a return information use fee to be imposed on every receipt creation institution, corresponding to frequency of use of the return information of the return information storing portion in every client system.

Since the return information is especially useful in this input support system, it is possible, by setting charges corresponding to the frequency of use of the return information, that a provider of this system charges objectively-valid fees to the respective receipt creation institutions as system users.

### Brief Description of Drawings

FIG. 1 is a block diagram showing a general configuration of a receipt creation support system according to one embodiment of the present invention.
FIG. 2 is a block diagram showing one example of a configuration of an application server provided to a server system of the receipt creation support system.
FIG. 3 is a block diagram showing one example of a configuration of a computer provided to a client system of the receipt creation support system.
FIG. 4 is a flow chart showing an operation procedure of the computer.
FIG. 5 is a flow chart showing an operation procedure of the application server.
FIG. 6 is an explanatory view showing a data input screen displayed on a display of the computer, and an example of inputted data.
FIG. 7 is an explanatory view showing an example of a return tag attached to a return receipt from an audit institution.
FIG. 8 is an explanatory view showing an example of contents of return information registered in an error adjustment DB server of the server system.
FIG. 9 is an explanatory view showing that the input data of FIG. 6 is checked on the basis of the return information shown in FIG. 8 and defective data are highlighted.
FIG. 10 is an explanatory view showing a piece of return information corresponding to the same case as the defective data highlighted in FIG. 9.
FIG. 11 is an explanatory view showing that the input data of FIG. 6 are revised on the basis of the return information of FIG. 10.
FIG. 12 is an explanatory view showing an example of display of return information in a case that use/no-use of return information is selectable.
FIG. 13 is an explanatory view showing an example of description data of a submitted receipt displayed on the data input screen, for creating a receipt to be re-submitted after a return.
FIG. 14 is an explanatory view showing that defective data that causes a return is selected by an operator.
FIG. 15 is an explanatory view showing that the defective data are revised by an operator to a piece of correct data.
FIG. 16 is an explanatory view showing that description data of a receipt for resubmission, which is created on the basis of revised data, are displayed in a data input screen.
FIG. 17 is an explanatory view showing an example of returns based on audit standards unique to each audit institution.
FIG. 18 is an explanatory view showing another example of returns based on audit standards unique to each audit institution.
FIG. 19 is a block diagram showing a configuration of an application server provided to a server system, in a receipt creation support system according to another embodiment of the present invention.
FIG. 20 is an explanatory view showing contents of return information presented on the basis of the return as shown in FIG. 17.
FIG. 21 is an explanatory view showing contents of return information presented on the basis of the return as shown in FIG. 18.
FIG. 22 is a flow chart showing a procedure of a process for determining regional uneven distribution of return information.
FIG. 23 is an explanatory view showing an example of contents of return information determined in its regional uneven distribution in accordance with the procedure shown in FIG. 22.
FIG. 24 is a block diagram showing a general configuration of a managerial decision request system according to still another embodiment of the present invention.
FIG. 25 is a block diagram showing an internal configuration of a server of the managerial decision request system.
FIG. 26 is a block diagram showing an internal configuration of a client computer of the managerial decision request system.
FIG. 27 is an explanatory view showing an example of an initial screen of a managerial decision request system displayed in the client computer.
FIG. 28 is an explanatory view showing an example of an input screen of the managerial decision request system.
FIG. 29 is an explanatory view showing an example of managerial decision request information accumulated in the managerial decision request system.
FIG. 30 is an explanatory view showing an example of a correction-directing screen displayed in the client computer.
FIG. 31 is an explanatory view showing another example of an input screen of the managerial decision request system.
FIG. 32 is an explanatory view showing another example of managerial decision request information accumulated in the managerial decision request system.

### Preferred Embodiment of the Invention

### (First embodiment)

Hereinafter, an embodiment of the present invention is described with reference to the attached drawings.

In this embodiment, a receipt creation support system used in a medical institution or the like is described as an example of the input support system according to the present invention.

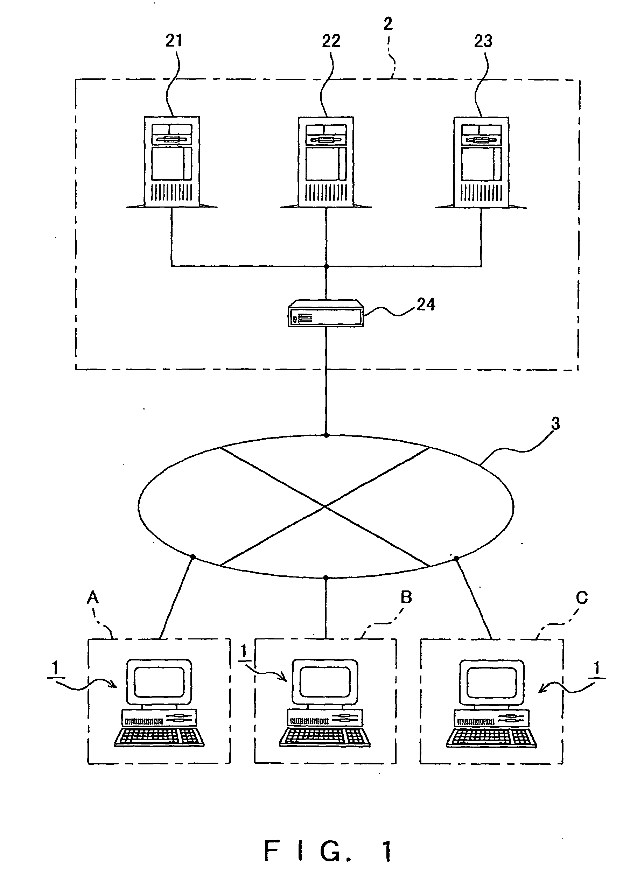

FIG. 1 is a block diagram showing a configuration of a receipt creation support system in this embodiment.

As shown in FIG. 1, the receipt creation support system has a configuration where computers 1 of client systems provided in respective medical institutions A, B, C... as receipt creation institutions and a server system 2 are connected through the Internet 3 (communications line).

Peripheral equipment such as printers is omitted from FIG. 1 illustrating the configuration of the client system of each medical institution, while only computers 1 as so-called desktop personal computers having displays and keyboards are shown. The configurations and scales of the client systems are not limited to the example shown in FIG. 1, but any suitable computer systems can be applied.

The server system 2 includes an application server 21, a receipt creation DB (database) server 22, an error adjustment DB server 23, and a router 24.

The application server 21 provides, through the Internet 3, a receipt creation service and a receipt check service to the computers 1 of the medical institutions A, B, C,... as subscribers of the receipt creation support system.

The receipt creation service in this embodiment denotes a service to provide on WWW (Word Wide Web) an input screen for data items required for creation of the receipt, allowing a user to input the data on the data input screen of the computers 1, receiving the inputted data, creating a receipt based on the received data, and transmitting the created receipt to the computers 1.

The receipt check service denotes a service of checking the description of a receipt for defects or errors before submitting the receipt to an audit institution. In this check, past return information accumulated in the error adjustment DB server 23 is used. The return information is collected from the medical institutions A, B, C,....

That is, the receipt creation support system concentrates, into this error adjustment DB server 23, information (return information) about the return that each medical institution receives from the audit institution, so that all the medical institutions entering this system share this return information, thereby avoiding returns caused by the same errors that any other medical institution has made.

The receipt creation DB server 22 has a master file to which the application server 21 refers at the time of creating a receipt, and a receipt file for saving the receipt created by the application server 21 for each medical institution. In the master file, a record including items of medical practices and the insurance scores for each of the medical items to which an insurance is applied are registered so as to be classified on the basis of the contents of the medical practices or the like.

In the error adjustment DB server 23, return information collected from the medical institutions A, B, C, ... is stored. The return information denotes information relating to a receipt returned from an audit institution to a medical institution. As described in detail later with reference to specific examples, it includes information such as description data (defective data) that causes the return and the way of correcting the defective data in accordance with instruction by means of a return tag.

The receipt creation support system will be described further for configurations of the respective portions and the operations.

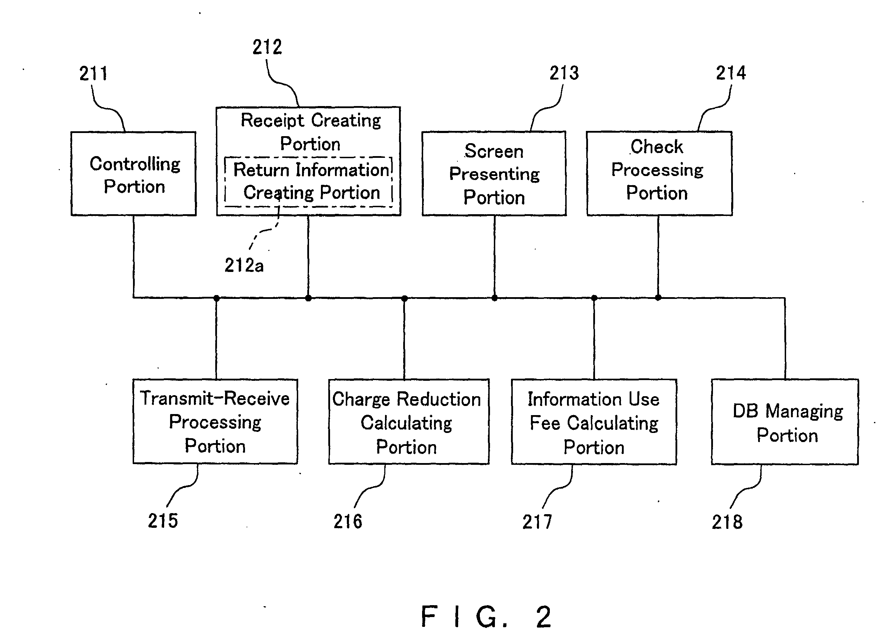

First, a configuration of the application server 21 is described in detail with reference to FIG. 2.

The application server 21 includes a controlling portion 211, a receipt creating portion 212, a screen presenting portion 213, a check processing portion 214, a transmit-receive processing portion 215, a charge reduction calculating portion 216, an information use fee calculating portion 217, and a data base (DB) managing portion 218.

The controlling portion 211 controls the operations of the entire application server 21 by transmitting directions to the respective portions of the application server 21. The transmit-receive processing portion 215 controls data communication with the computers 1 through the Internet 3. The DB managing portion 218 has functions to search the receipt creation DB server 22 and the error adjustment DB server 23 so as to acquire required data, and register the data in these databases.

The receipt creating portion 212 is a block for realizing the above-described receipt creation service. The receipt creating portion 212 has also a function of creating a receipt for resubmission by correcting defective data of the returned receipt. Furthermore, the receipt creating portion 212 has a return information creating portion 212a for automatically creating return information to be registered in the error adjustment DB server 23 at the time of creating the receipt for resubmission.

The screen presenting portion 213 provides to the computer 1 of each medical institution through the transmit-receive processing portion 215 and the Internet 3 the data input screen defined by a page-description language such as a HTML language, when there is a request for a data input screen for creating a receipt from the computer 1 through the Internet 3.

The check processing portion 214 is a block for providing the above-described receipt check service. The check processing portion 214 has two kinds of processing modes of (1) a batch process to perform a batch check for all the description data in one or plural receipt(s) that have been subjected to a substantially thorough data input, and (2) a real-time process of sequentially operating with the above-described receipt creation service and checking the data every time data are inputted during creation of the receipt. The computers 1 serve to select which processing mode will be used for employ the receipt check service. The check processing portion 214 operates according to the selection in the computer 1.

The charge reduction calculating portion 216 and the information use fee calculating portion 217 are blocks for calculating system use fees that the respective medical institutions A, B, C, ... receiving the receipt creation service and the receipt check service are to pay to the manager of the server system 2 as a service provider.

Each of the medical institutions can enjoy discounts of the system use fee, corresponding to, e.g., the number of the subjects of return information that it provided to the server system 2 and the frequency of providing the return information. The charge reduction calculating portion 216 calculates the discount rate. The system use fee includes an information use fee calculated corresponding to the frequency of use of the return information of the error adjustment DB server 23. The information use fee calculating portion 217 calculates this information use fee.

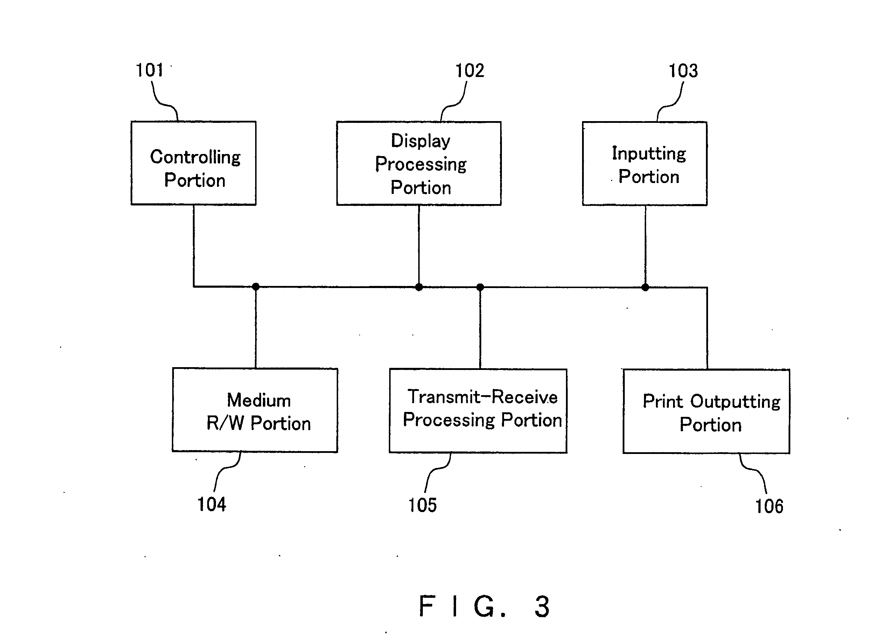

Next, the configuration of a computer 1 of any of the medical institutions A, B, C, ... is described below by referring to FIG. 3.

The computer 1 has a controlling portion 101, a display processing portion 102, an inputting portion 103, a medium R/W (Read/Write) portion 104, a transmit-receive processing portion 105, and a print outputting portion 106.

The controlling portion 101 controls the entire operations of the computer 1 by sending directions to the respective portions. The display processing portion 102 has a browsing function, and it controls the display of the computer 1 so as to perform a process of displaying the data input screen acquired from the application server 21. The inputting portion 103 accepts data input into the data input screen. The data input is carried out by the operator of the computer 1 by using an input device (a keyboard, various pointing devices, or the like).

The medium R/W (Read/Write) portion 104 performs writing of the created receipt into an information recording medium and reading of a receipt from an information recording medium in which the receipt is recorded. For the information recording medium, a hard disk, and also a floppy disk, MO, CD-RW, DVD-RAM or the like can be used. In each medical institution, suitable media can be selected corresponding to the quantity or the like of the receipts to be processed.

The transmit-receive processing portion 105 controls data communications to the server system 2 through the Internet 3. The print outputting portion 106 performs a process of outputting a created receipt, a list of a result of the check process for the receipt or the like, with regard to a printer (not shown) connected to the computer 1.

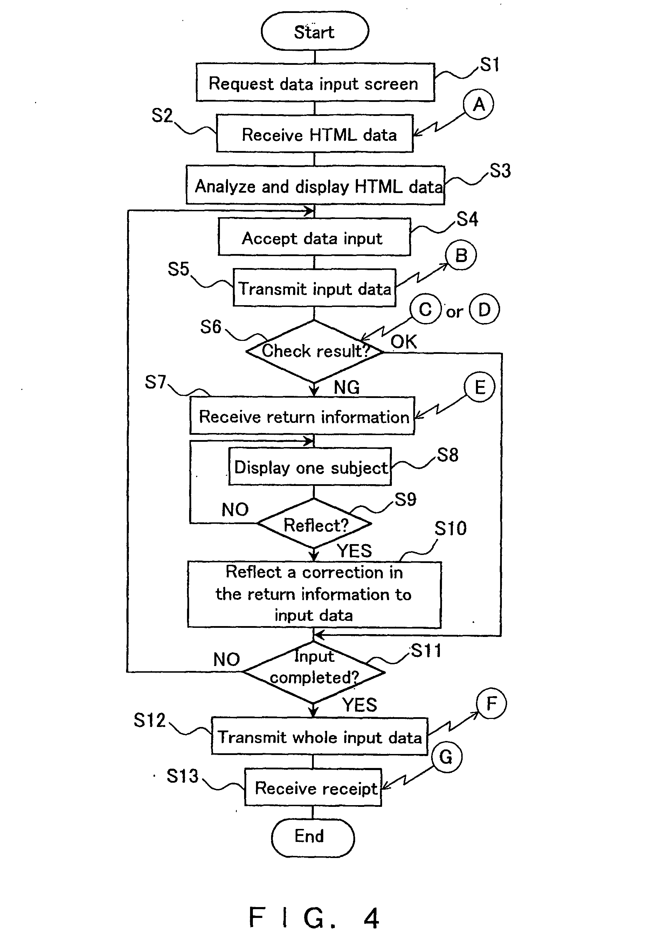

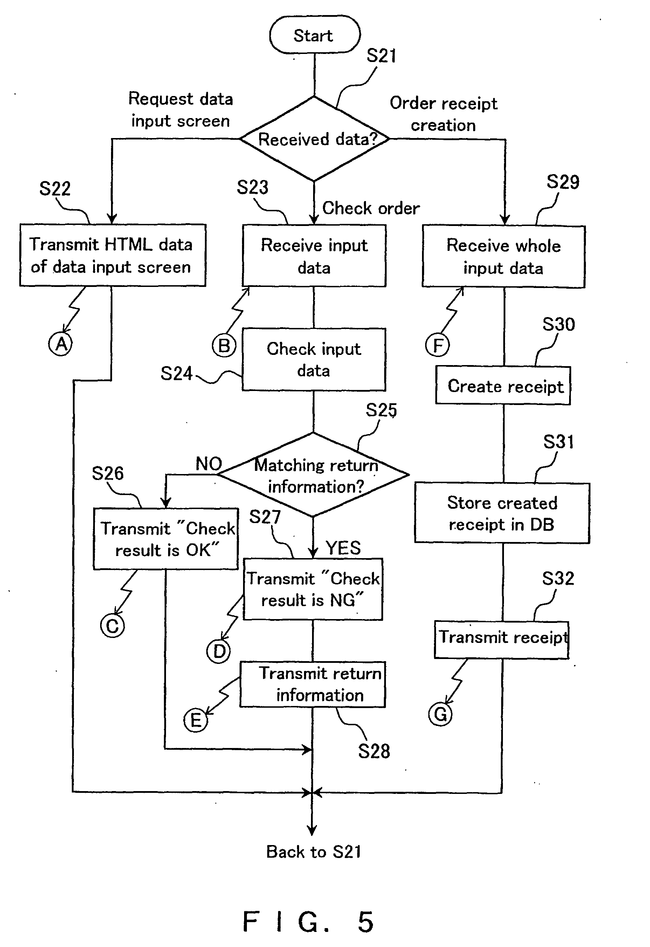

Next, an operation of the receipt creation support system will be described in detail by using specific examples. The operation procedure of the computer 1 is shown in FIG. 4, and the operation procedure of the application server 21 in the server system 2 is shown in FIG. 5.

In the case of creating receipts in any of the medical institutions A, B, C,..., first, the controlling portion 101 of the computer 1 asks the application server 21 for a data input screen through the transmit-receive processing portion 105 in order to allow the display to indicate the data input screen for inputting data required for creation of the receipts (step S1 in FIG. 4).

In the application server 21, when the transmit-receive processing portion 215 receives the request, the controlling portion 211 proceeds with the control from step S21 to step S22 (see FIG. 5). Then, in step S22, under a control of the controlling portion 211, the screen presenting portion 213 sends HTML data defining the data input screen to the transmit-receive processing portion 215, and the transmit-receive processing portion 215 transmits the HTML data to the requesting computer 1. When the transmission is completed, the controlling portion 21 puts the control back to step S21.

In FIG. 4 and FIG. 5, various kinds of data transmitted and received between the computer 1 and the application server 21 are indicated with signs of A to G. For example, the sign A shown in FIG. 5 represents HTML data transmitted from the application server 21 to the computer 1 due to a process in step S22, and the HTML data are received by the computer 1 in step S2 in FIG. 4.

In the computer 1, upon receiving the HTML data in step S2, the display processing portion 102 analyzes the received HTML data under a control of the controlling portion 101 and displays, as a data input screen, in the display (step S3). Afterwards, when the operator of the computer 1 performs input of data required for creating a receipt with respect to this data input screen, the inputting portion 103 accepts this data input (step S4).

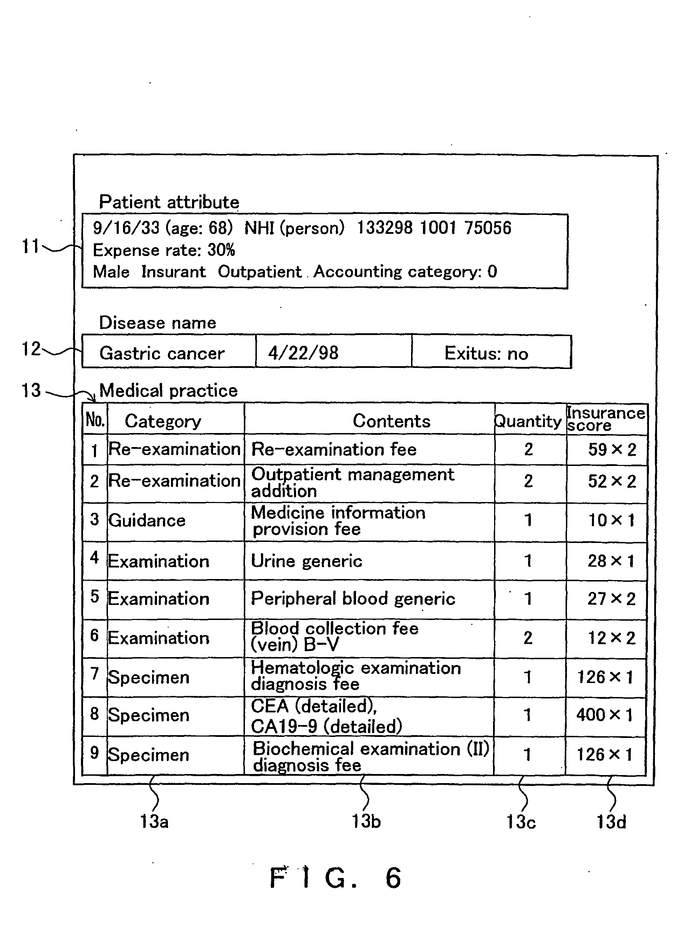

Here, an example of the data input screen is shown in FIG. 6, and the way of inputting data into the data input screen is described below. FIG. 6 shows an example of the screen in the state that all required data have been inputted. As shown in FIG. 6, a data input screen has a patient attribute column 11 for inputting data relating to patient attribute, a disease name column 12 for inputting a name of the patient's disease, and a medical practice column 13 for inputting medical practices conducted within the month with respect to the patient.

The medical practice column 13 is divided into a category column 13a for inputting a category of the medical practice, a medical item column 13b for inputting the contents of the medical practices, a quantity column 13c for inputting the quantity (or the number) of the medical practices, and an insurance score column 13d for inputting the provided insurance scores.

Though not shown in FIG. 6, in each of the respective columns described above, for example, a pulldown menu or the like is set. Options displayed by the pulldown menu are created from the master file of the receipt creation DB server 22.

For example, in the case of the category column 13a of the medical practice column 13, as partly shown in FIG. 6, names of categories registered in the master file of the receipt creation DB server 22, i.e., categories of medical practices to which insurances are applicable, such as "initial medical examination", "re-examination", "guidance", "home care", "compounding", "examination", "specimen", and "diagnosis", are displayed as options in the pulldown menu. Accordingly, the operator of the computer 1 can perform data input easily by only selecting a suitable category name from the pulldown menu.

A method of inputting into the category column 13a is not limited to the selection from the pulldown menu, but various other methods can be applied. Another available example is a method of inputting by using category codes that have been decided previously corresponding to the respective category names. In this case, category codes corresponding to the respective category names (for example, a category code "A000" is applied to a category name "initial medical examination") are registered previously in the master file of the receipt creation DB server 22. When the operator of the computer 1 inputs the category code "A000" in the category column 13a by means of a keyboard or the like, a category name "initial medical examination" corresponding to this category code is acquired from the master file of the receipt creation DB server 22, and displayed in the category column 13a of the data input screen.

When an input into the category column 13a is performed, under the control of the controlling portion 101, the inputting portion 103 accepts the input data (category name), and the transmit-receive processing portion 105 transmits the input data to the application server 21. In the application server 21, under a control of the controlling portion 211, the category name received by the transmit-receive processing portion 215 is sent to the DB managing portion 218. The DB managing portion 218 acquires, from the receipt creation DB server 22 under the control of the controlling portion 211, the names of all the medical items corresponding to the category names.

The medical item names acquired by the DB managing portion 218 are sent to the computer 1, and displayed by the display processing portion 102 as options in the pulldown menu of the medical item column 13a of the data input screen. Thereby, the operator of the computer 1 can perform the data input easily by only performing selection from the menu. The method of inputting into the medical item column 13b, similar to the data input into the category column 13a, is not limited to a selection from the pulldown menu.

Next, when the operator of the computer 1 inputs the quantity (the number) of the medical practices into the quantity column 13c, under a control of the controlling portion 101, the data inputted into the category column 13a, the medical item column 13b and the quantity column 13c are transmitted by the transmit-receive processing portion 105 to the application server 21.

In the application server 21, under a control of the controlling portion 211, the receipt creating portion 212 receives these, and the DB managing portion 218 acquires an insurance score of the medical practice from the master file of the receipt creation DB server 22. The acquired insurance score is transmitted to the computer 1, and displayed in the insurance score column 13d by the display processing portion 102. That is, in the receipt creation support system, the operator of the computer 1 is not required to investigate the insurance scores of the respective medical practices by himself for the purpose of inputting.

As mentioned above, when the operator of the computer 1 inputs data into the data input screen, under the control of the controlling portion 101, the input data are sent from the inputting portion 103 to the transmit-receive processing portion 105. The transmit-receive processing portion 105 transmits the input data (B) to the application server 21 under a control of the controlling portion 101 (step S5). The transmit-receive processing portion 105 adds an indication representing a check order at the head of the input data to be transmitted.

On the other hand, when the application server 21 receives from the computer 1 the input data with the added indication, the controlling portion 211 recognizes that there is an order from the computer 1 for checking the data, and proceeds with the control from step S21 to step S23, and allows the transmit-receive processing portion 215 to receive the input data. The controlling portion 211 sends the received input data to the check processing portion 214 and allows to perform the process of checking the input data (steps S24-S28).

Hereinafter, the check process carried out by the check processing portion 214 is described below in detail, based on a specific data example as shown in FIG. 6. This description is predicated on the data example shown in FIG. 6 including defective data, and information of a return that has been conducted with respect to a receipt including defective data similar to the above defective data is registered in the error adjustment DB server 23.

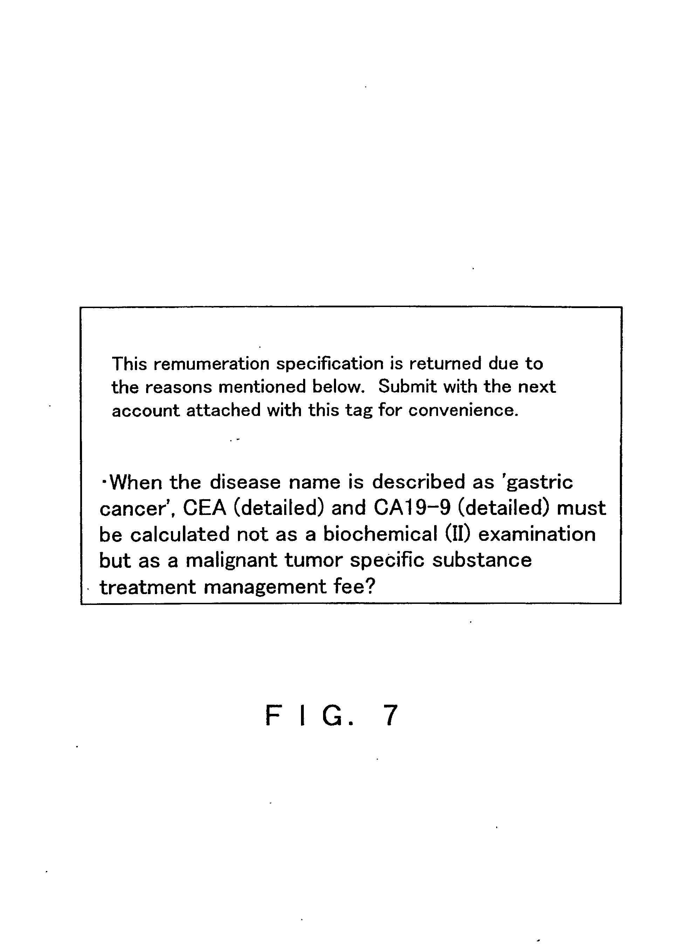

The example of data shown in FIG. 6 includes defective data. Therefore, a receipt created directly from the data and submitted will be returned with a return tag of a message as shown in FIG. 7 as a result of an audit at an audit institution. That is, when the disease name is determined as "gastric cancer", examinations of CEA (detailed), CA19-9 (detailed) cannot be calculated as examination fees, but they must be calculated as management fees. A return tag shown in FIG. 7 includes this message provided by the audit institution.

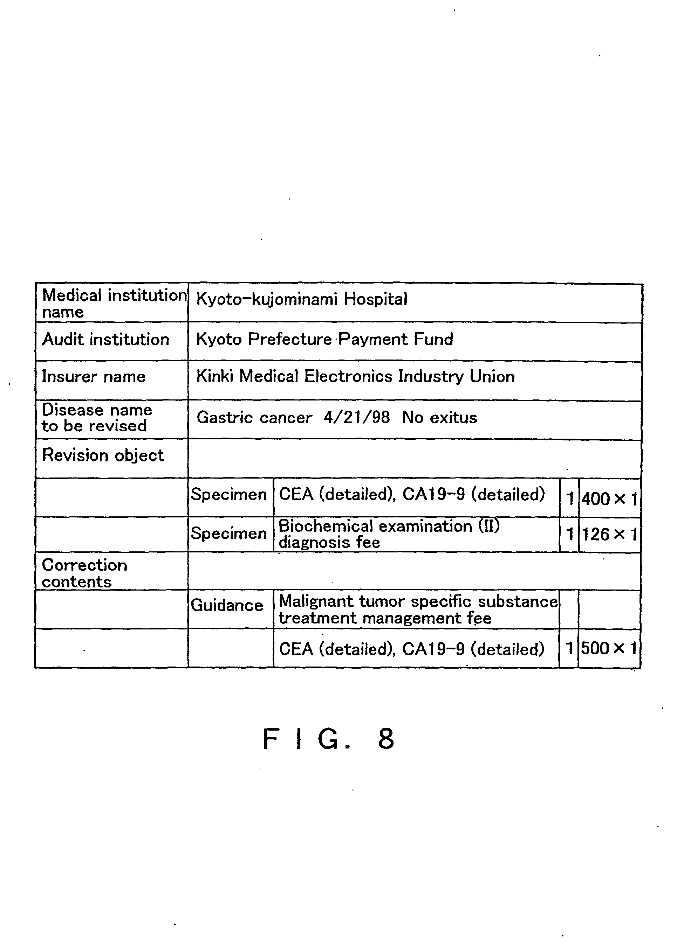

As mentioned above, in this explanation, one or more of the medical institutions A, B, C,... has experienced a return based on similar defective data, and based on information provided by the medical institution, the return information is registered in the error adjustment DB server 23. A specific example of the return information is shown in FIG. 8. Registration procedures or the like of the return information in the error adjustment DB server 23 will be explained later.

As shown in FIG. 8, the return information includes a name of a medical institution receiving the return (a medical facility name in FIG. 8), an audit institution name, an insurer name of a receipt to be returned, a disease name described in the receipt (disease name to be revised), contents of defective data that become an object of the revision due to the return (revision object), and correction contents with respect to the defective data.

The return information shown as a specific example in FIG. 8 represents that a "Kyoto Kujominami Hospital" as one of the medical institutions A, B, C, ... received a return based on similar defective data (examinations for CEA (detailed) and CA19-9 (detailed) in a case of a disease name of gastric cancer), and the hospital corrected "biochemical examination (II) diagnosis fee" to "malignant tumor specific substance treatment management fee" and re-submitted it, which was admitted.

In step S24, the check processing portion 214 sends a direction to the DB managing portion 218, and compares input data to be checked and return information that is registered in the error adjustment DB server 23 so as to perform a check of the input data. When return information matching the input data exists in the error adjustment DB server 23, this means that the input data may be returned, and thus the check result will be "NG".

The matching of the input data and the return information is determined depending on whether a piece of return information that includes in its return object item the same data as the input data that exists in the error adjustment DB server 23. Here, a combination of the eighth and ninth data in the input data shown in FIG. 6 matches data in the revision object items in the return information shown in FIG.8. Therefore, the determination result in step S25 becomes YES, and the check result becomes "NG".

When the determination result in step S25 is YES, the controlling portion 211 sends a direction to the transmit-receive processing portion 215, and transmits to the computer 1 an indication (D) representing that the check result of the input data is "NG" (step S27). The controlling portion 211 further allows the transmit-receive processing portion 215 to transmit to the computer 1 return information (E) extracted from the error adjustment DB server 23 due to its matching with the input data to be checked (step S28).

When return information matching the input data to be checked does not exist in the error adjustment DB server 23, an indication (C) representing "OK" as a result of the check of the input data is transmitted from the transmit-receive processing portion 215 to the computer 1 (step S26).

When the computer 1 receives an indication (C or D) representing the check result of the input data, and when the indication represents "NG", the computer 1 receives the return information transmitted with the indication (step S7). Then, under a control of the controlling portion 101, the display processing portion 102 displays the input data to be checked in a highlighted state, and indicates, in the display, return information received in step S7 (step S8).

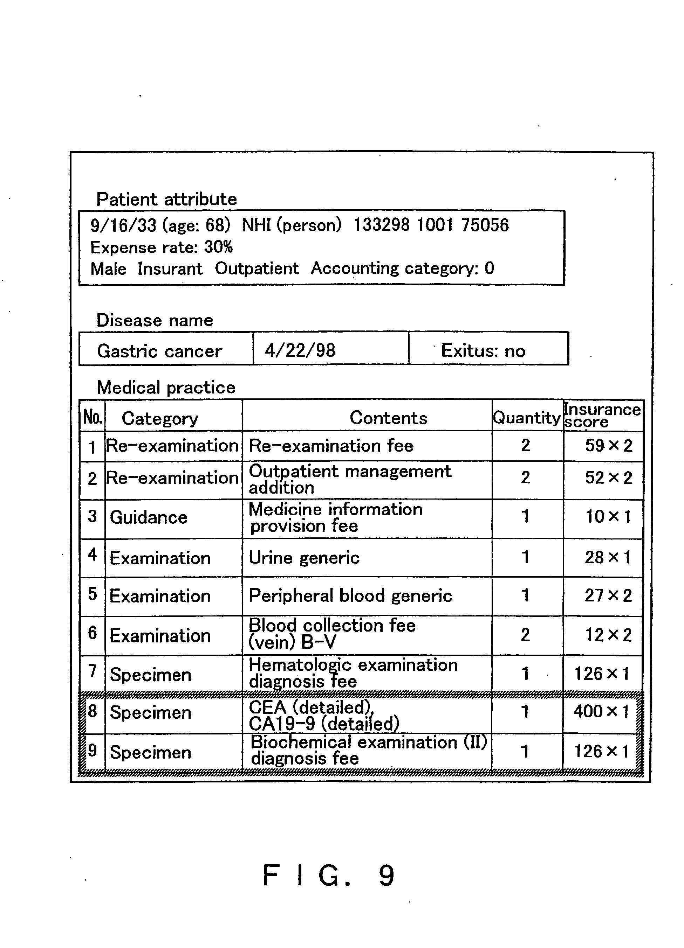

FIG. 9 shows an example of a highlighted display of input data to be checked. In the example, the input data is encircled with a thick frame for highlighting. In fact, highlighted displays are not limited thereto, but any suitable methods can be taken as long as the data can be distinguished from the remaining data.

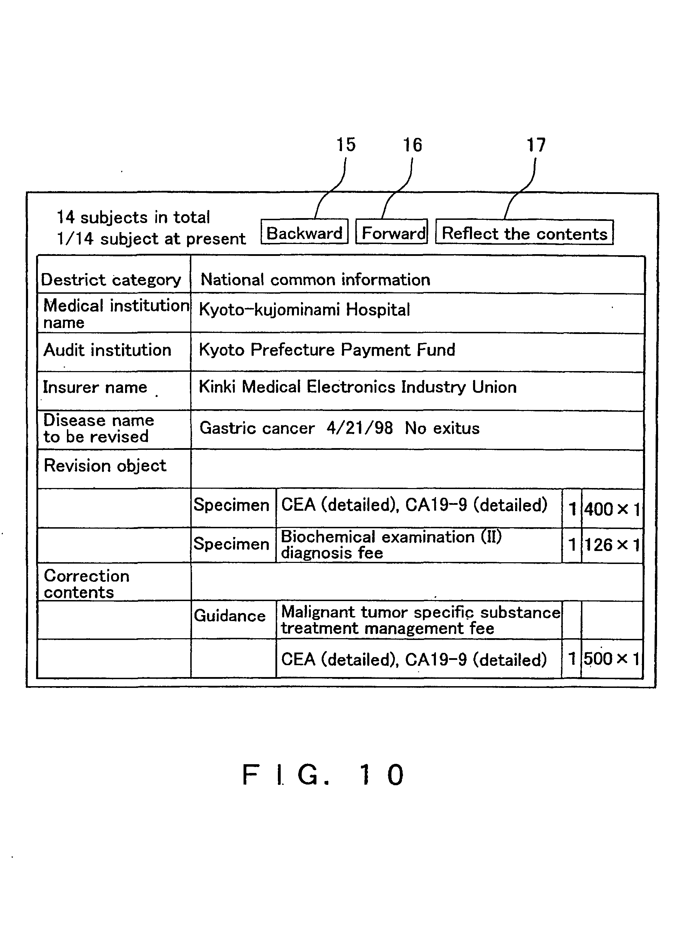

FIG. 10 shows an example of displaying return information in step S8. This example includes 14 subjects of return informations extracted from the error adjustment DB server 23 due to their matching with the input data. The first return information is shown in this figure. This screen can be displayed, on a display of the computer 1, in a window separated from the data input screen. When the operator wants to refer to any other return information, he can display the other return information by clicking the button 15 or button 16 indicated as "backward" or "forward" on this screen.

The information use fee calculating portion 217 of the application server 21 has a counter for counting the number of the return information use cases in every medical institution, and it adds '1' to a counter value of the medical institution every time one subject of return information is displayed on the display of the computer 1. And, for example, a return information use fee to be charged to each medical institution is calculated every month, based on the counter value.

When the operator clicks a button 17 indicating "Reflect this content" on the screen (YES in step S9), the inputting portion 103 accepts this and reflects the return information displayed on this screen to the input data on the data input screen (step S10). That is, the inputting portion 103 replaces the input data highlighted on the data input screen by the data in the correction contents item in the return information.

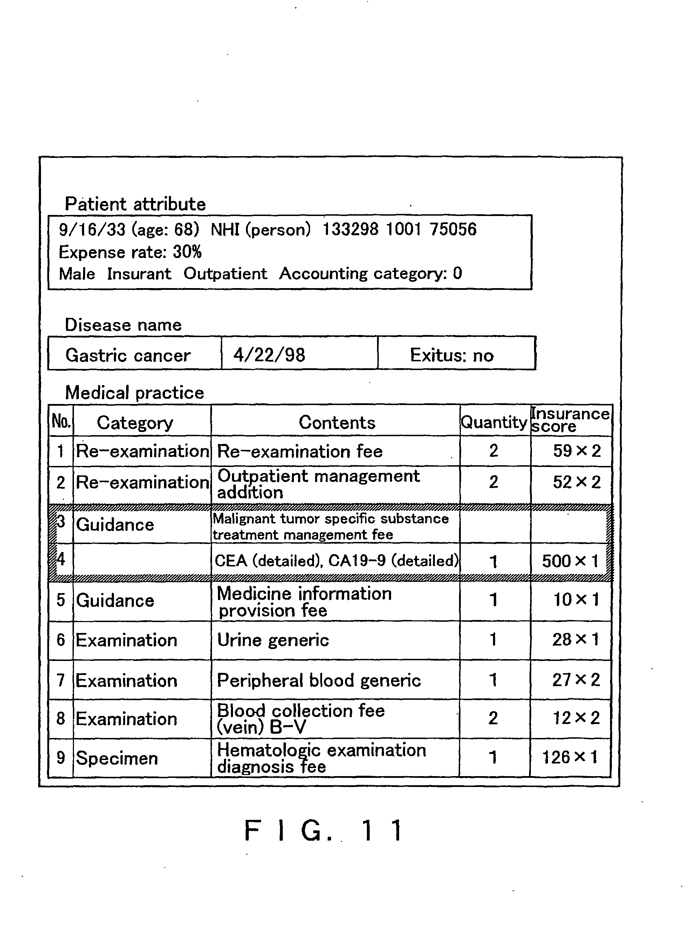

For example, on the screen shown in FIG. 10, when the button 17 is clicked, the input data highlighted in FIG. 9 are replaced by the data in the correction contents item displayed in FIG. 10, so that the display condition of the data input screen becomes as shown in FIG. 11. In FIG. 11, the part encircled with a thick frame denotes the replaced data.

As mentioned above, based on the return information of the error adjustment DB server 23, a check of the input data on the data input screen and revision of defective data are performed. This enables creation of a receipt that will not be returned for at least the same reason as past returns.

Subsequently, in the computer 1, under the control of the controlling portion 101, the display processing portion 102 displays for the operator a message for confirming whether inputting of the whole data has been completed or not, and when the input will be continued (NO in step S11), it goes back to step S2. When completion of the input is confirmed (YES in step S11), under the control of the controlling portion 101, the whole data of the data input screen are sent from the inputting portion 103 to the transmit-receive processing portion 105. The transmit-receive processing portion 105 adds to the head an indication representing a receipt creation order, and transmits the whole input data (F) of the data input screen to the application server 21 (step S12).

When the application server 21 receives the receipt creation order from the computer 1, the controlling portion 211 proceeds with the control from step S21 to step S29, and receives, by means of the transmit-receive processing portion 215, the whole input data (F) transmitted from the computer 1.

The received whole input data are sent from the transmit-receive processing portion 215 to the receipt creating portion 212. Based on the received whole input data, the receipt creating portion 212 creates a receipt in accordance with a fixed form (step S30). The created receipt is sent from the receipt creating portion 212 to the DB managing portion 218 and the transmit-receive processing portion 215. The DB managing portion 218 sends this receipt to the receipt creation DB server 22 so as to allow storage of this receipt in a receipt file of the medical institution (step S31). The transmit-receive processing portion 215 sends this receipt (G) back to the computer 1 as a sender of the input data (step S32).

In the computer 1, the transmit-receive processing portion 105 receives the receipt (G) transmitted from the application server 21 (step S13). This receipt is written in the information recording medium by the medium R/W portion 104, and stored.

As a result of the processes, the receipt creation support system can provide a receipt creation service to each of the medical institutions A, B, C,..., and it also can provide a receipt check service based on the past return information accumulated in the error adjustment DB server 23.

In the example described here, the created receipt is stored in both the receipt creation DB server 22 and the information recording medium dealt with by the medium R/W portion 104, i.e., stored in the server system 2 and the medical institution. This is not to be considered limiting, but the receipt can be stored in any one of them. Furthermore in the computer 1, the receipt received in step S13 can be sent to the print outputting portion 106 and outputted by a printer connected to the computer 1.

When the controlling portion 101 of the computer 1 proceeds with the control to step S5 every time one subject of data is inputted in the medical practice column 13 of the data input screen in the above-mentioned step S4 so as to transmit the data to the application server 21 to be subjected to a check process, a data check can be performed in real time, and defects caused by erroneous input can be prevented.

The above-described example will be used for the following explanation. At a time that the eighth data in the data input screen shown in FIG. 6 are inputted (i.e., before inputting the ninth data), a check is performed for the data, so that return information as shown in FIG. 8 is presented as return information including in the revision object item a piece of data same as indicated in the contents column 13b. Therefore, when the operator of the computer 1 refers to the return information, he will recognize that "CEA (detailed), CA19-9 (detailed)" must be calculated not as "biochemical examination (II) diagnosis fee" but as "malignant tumor specific substance treatment management fee", and thus, defects caused by erroneous input can be prevented.

On the other hand, the controlling portion 101 of the computer 1 proceeds with the control to step S5 at the time a certain amount of data are inputted in step S4 (for example, whole data for a patient, or whole data for whole patients), so that a certain amount of input data are subjected to a check process in a batch (a batch process). Or, the result of the check process can be provided in a listed form.

In the case of this batch process, the defects cannot be checked at the same time of inputting data. However, it has an advantage that the overhead for transmitting and receiving data is reduced in comparison with the above-mentioned real time check process, and the receipt creation time can be shortened as a whole.

It is possible to select arbitrarily from the computer 1 whether the check process with respect to the input data is performed in the above-described real-time process or a batch process.

The receipt creation support system can import receipt information created by an external system (for example, an electronic chart system of the medical institutions A, B, C,...), and performs a receipt check process. In this case, the receipt information created at the external system can be imported by being received at the transmit-receive processing portion 215 of the application server 21 through the Internet 3.

Alternatively, each medical institution can record a receipt created by using the electronic chart system or the like on an information recording medium and send it to the manager of the server system 2, so that in the server system 2, the application server 21 acquires the receipt information from the information recording medium.

A result of the check process for the imported receipt can be noticed to each medical institution through the Internet 3, or it can be sent to each medical institution after being compiled in a list form.

In some cases, the return information that is extracted from the error adjustment DB server 23 and presented as a result of the process of checking the data may include return information that cannot be a cause of the return for some medical institutions. Regarding this, when the return information is presented in the computer 1 of the medical institution, selection about whether to use the return information hereinafter can be carried out, and also the selection result can be registered in the error adjustment DB server 23. According to this configuration, useless return information will not be displayed, thereby facilitating the user's convenience.

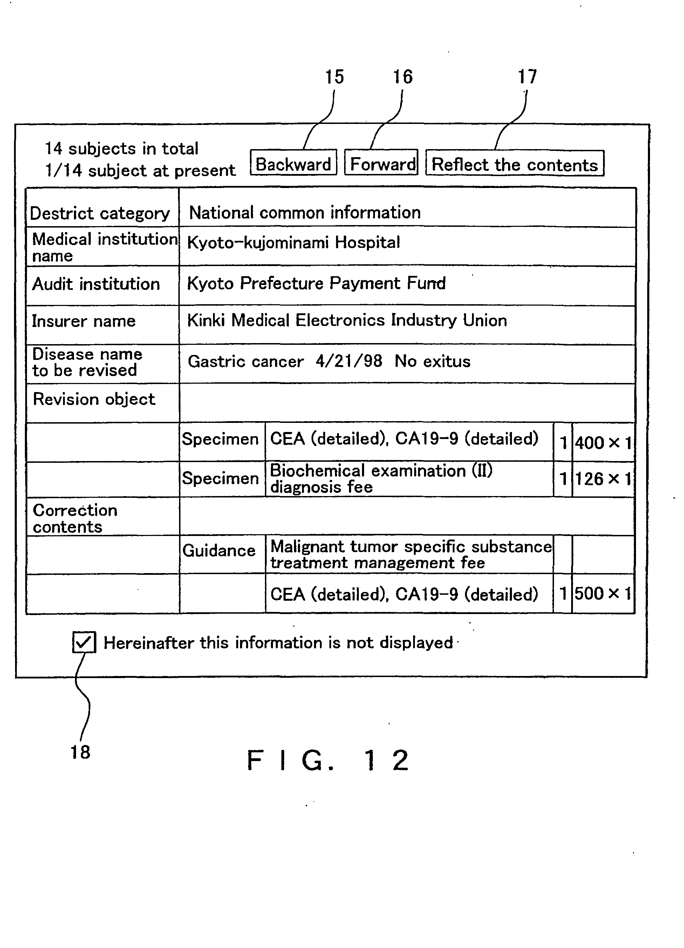

In this case, as shown in FIG. 12, at the time of displaying return information on the display of the computer 1, a check box 18 is provided for selectively inputting whether to use the return information hereinafter. Then, in the return information recorded in the error adjustment DB server 23, a use flag item representing whether to use the return information is provided for each medical institution. When a check is inputted in the check box 18 at the time of presenting return information to a medical institution, a flag representing "no-use" is set ON in the use flag item of the medical institution regarding the return information. Thereafter, when the "no-use" flag is ON, the return information will not be presented to the medical institution. Thereby, required return information can be presented to each medical institution selectively and exclusively.

The following explanation is about registration of return information in the error adjustment DB server 23 of the receipt creation support system.

As mentioned above, information (return information) relating to past returns, collected from the medical institutions A, B, C,... as subscribers of this receipt creation support system, is accumulated in the error adjustment DB server 23.

In the receipt creation support system, generally, there are two kinds of methods for collecting return information from the respective medical institutions. A first method is that when there is a return from an audit institution, in a medical institution an operator inputs the contents of the return and transmits them to the server system 2. A second method is to create return information automatically at the time of creation of a receipt for resubmission, which is performed at the time of a return.

In the case of the first method, the operator of the computer 1 transmits the return information to the application server 21 by using e-mail or the like, and the DB managing portion 218 of the application server 21 registers this return information in the error adjustment DB server 23.

In this case, the charge reduction calculating portion 216 of the application server 21 identifies which medical institution has transmitted the return information, and reduces the system use fee to be charged to each medical institution, depending on the number of subjects or frequency of return information provided from the medical institution. Accordingly, each medical institution is motivated to provide return information.

However, since the first method requires input work by the operator, a thorough collection of the return information would be difficult even by encouraging provision of information by, for example, reducing the system use fee depending on the number of subjects or frequency of providing the return information.

Therefore in the second method, the return information is created automatically at the time of creation of a receipt for resubmission, which is performed inevitably when there is a return from the audit institution.

For this purpose, the application server 21 has a return information creating portion 212a that creates return information from the unrevised and revised receipt description data, at the time of creating the receipt for resubmission at the receipt creating portion 212.

In the following, a procedure for a process of creating return information by the return information creating portion 212a will be described with reference to a specific example.

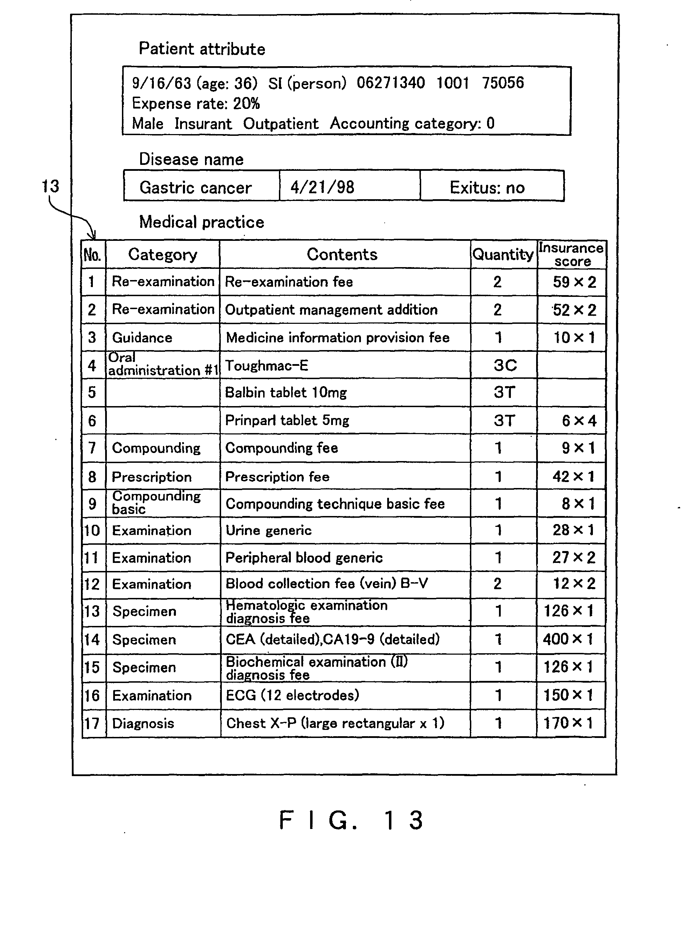

When there is a return from the audit institution, each medical institution revises the receipt and creates a receipt for resubmission. First, in the computer 1 of the medical institution, the returned receipt is indicated by the display. At this time, the receipt is read out from the master file of the receipt creation DB server 22 at the server system 2 and transmitted to the computer 1, and displayed on the data input screen by the display processing portion 102 as shown in FIG. 13.

In the example shown in FIG. 13, a combination of the 14^{th} and 15^{th} data in the medical practice column 13 makes a return reason, and the return tag is the same as shown in FIG. 7.

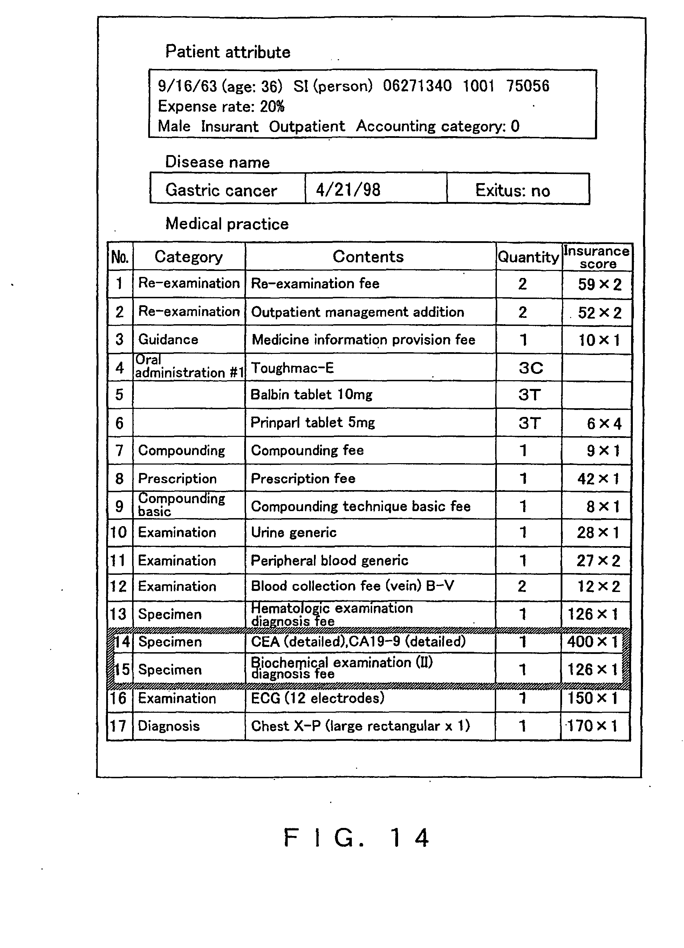

The operator of the computer 1 performs a revision with respect to this data input screen, in accordance with the contents pointed with the return tag. First, the operator selects the data part pointed with the return tag as data to be revised. FIG. 14 shows that the data to be revised are selected by the operator. Here, the data to be revised, which are selected by the operator, are sent from the inputting portion 103 to the transmit-receive processing portion 105, and further sent to the receipt creating portion 212 of the application server 21. At this time, the return information creating portion 212a of the receipt creating portion 212 sets the data to be revised in a revision object item for return information that will be newly created.

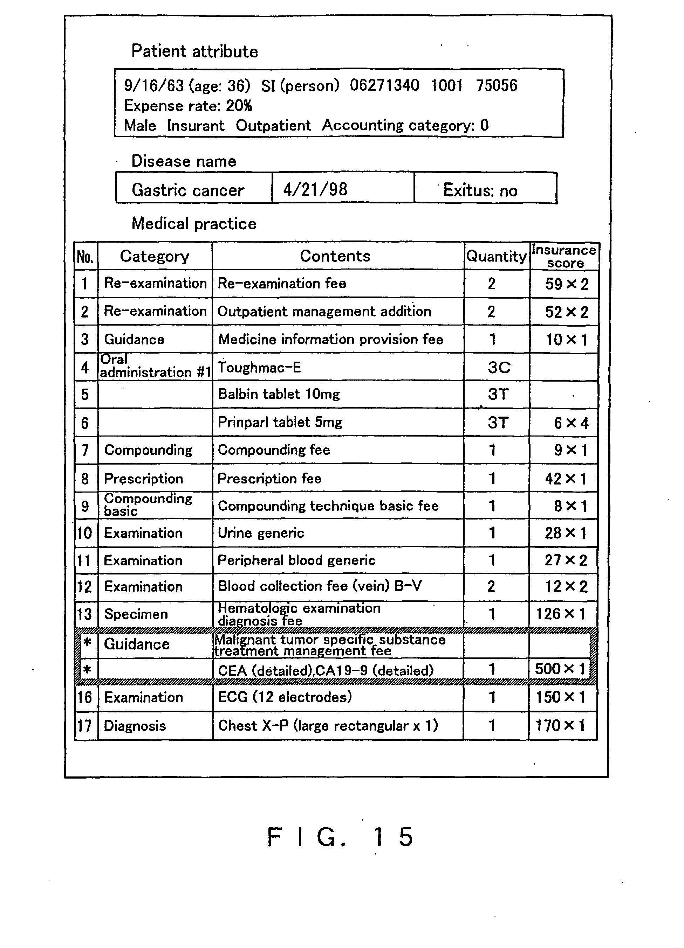

Next, the operator performs a revision input by deleting the data to be revised and rewriting it to correct data. The data newly inputted in place of the deleted data to be revised are sent to the receipt creating portion 212 of the application server 21 in a similar manner. At this time, the return information creating portion 212a of the receipt creating portion 212 sets the data to be revised in a correction contents item for the return information that will be newly created. FIG. 15 shows revision input performed by the operator. At the time of deleting the data to be revised, the number of the data is displayed as "*".

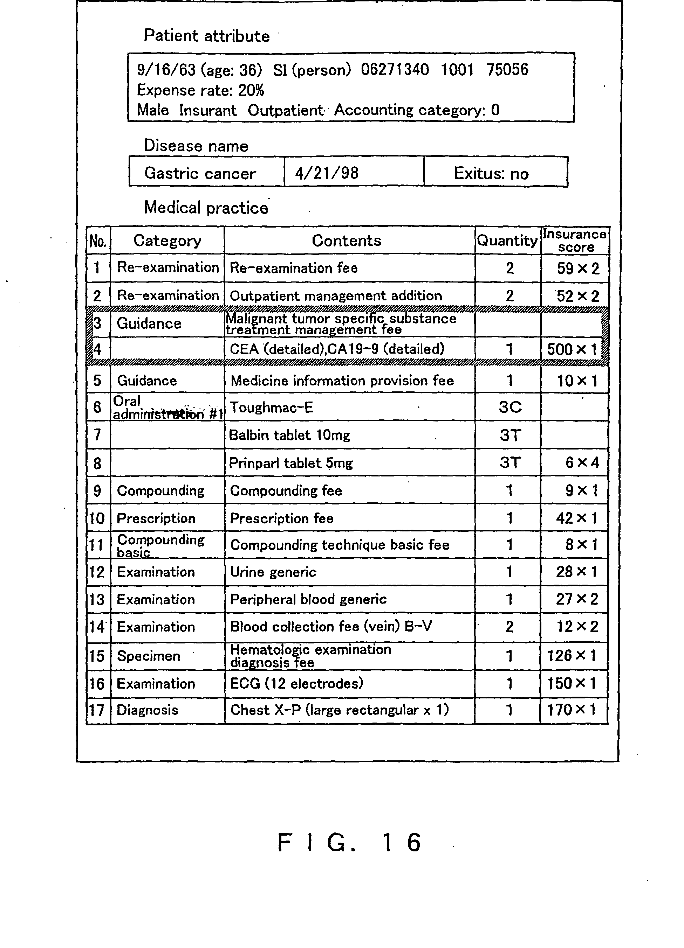

When the operator completes the revision input, the receipt creating portion 212 creates a receipt for resubmission on the basis of the revised data. The result is transmitted to the client system 1, and the display condition of the data input screen becomes as shown in FIG. 16. In FIG. 16, the part encircled with a thick line and highlighted denotes corrected data.

The return information creating portion 212a of the receipt creating portion 212 sets the patient attribute and the disease name of the created receipt for resubmission, as a patient attribute and a disease name of return information that will be newly created. Through these processes, the return information as shown in FIG. 8 is created by the return information creating portion 212a. This return information is sent from the return information creating portion 212a to the DB managing portion 218, and registered by the DB managing portion 218 in the error adjustment DB server 23 so as to form return information that is shared by all of the medical institutions A, B, C,....

As described above, since the return information is created automatically by the return information creating portion 212a at the time of creating the receipt for resubmission, information relating to all returns can be collected thoroughly into the error adjustment DB server 23. Thereby, the accuracy of the receipt check process can be improved.

### (Second Embodiment)

Another embodiment according to the present invention will be explained below by referring to the attached drawings. Similar to the first embodiment, an input support system of this embodiment refers to a receipt creation support system used in a medical institution or the like, and a variation of the system of the first embodiment.

The Fund or the Federation to which receipts are submitted have offices at respective prefectures in Japan including Hokkaido and the Tokyo Metropolis, and an audit board at each office functions as an audit institution. For this reason, audit standards may vary in the details among the prefectures.

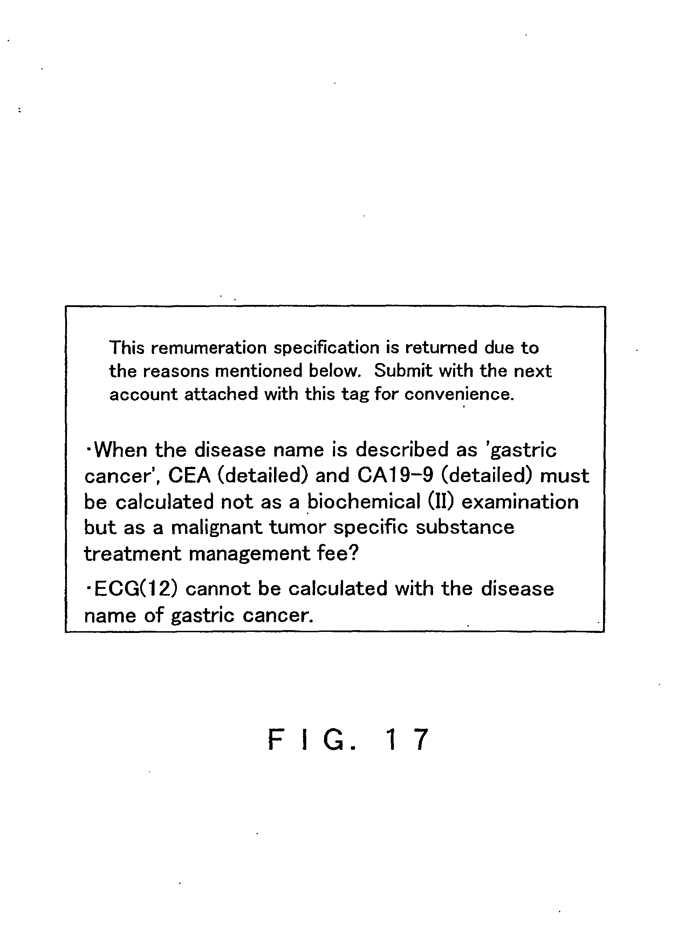

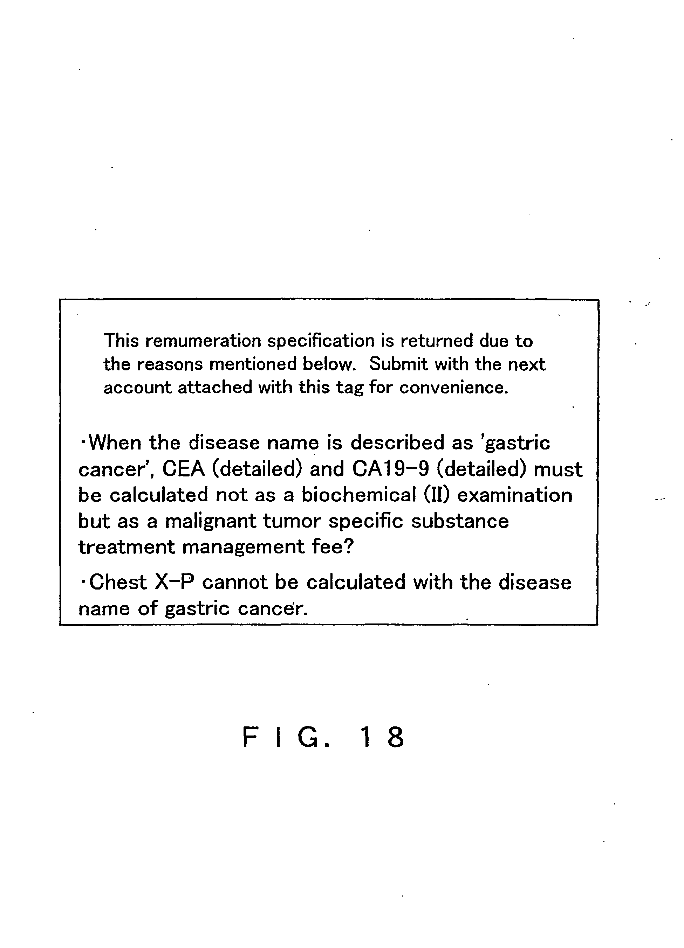

The following explanation is about an example of data shown in FIG. 13 referred to in the first embodiment. When a receipt based on the same data was submitted in a prefecture (XX prefecture), the 16^{th} data in the medical practice column 13 was determined also having a return reason, and the receipt was returned with a return tag as shown in FIG. 17. On the other hand, when a receipt based on the same data as described above was submitted in another prefecture (YY prefecture), the 16^{th} data in the medical practice column 13 was not a return reason, but the 17^{th} data was regarded as having a return reason, and the receipt was returned with a return tag as shown in FIG. 18. In the audit in the XX prefecture, the 17^{th} data were regarded as not having a return reason.

Therefore, the receipt creation support system of this embodiment classifies return information collected from medical institutions nationwide on a district-to-district basis, and performs the receipt check process of each medical institution on the basis of the return information of the district where the medical institution exists. Namely, it aims to improve the accuracy of the check process by enabling check suitable checking for the local audit standards.

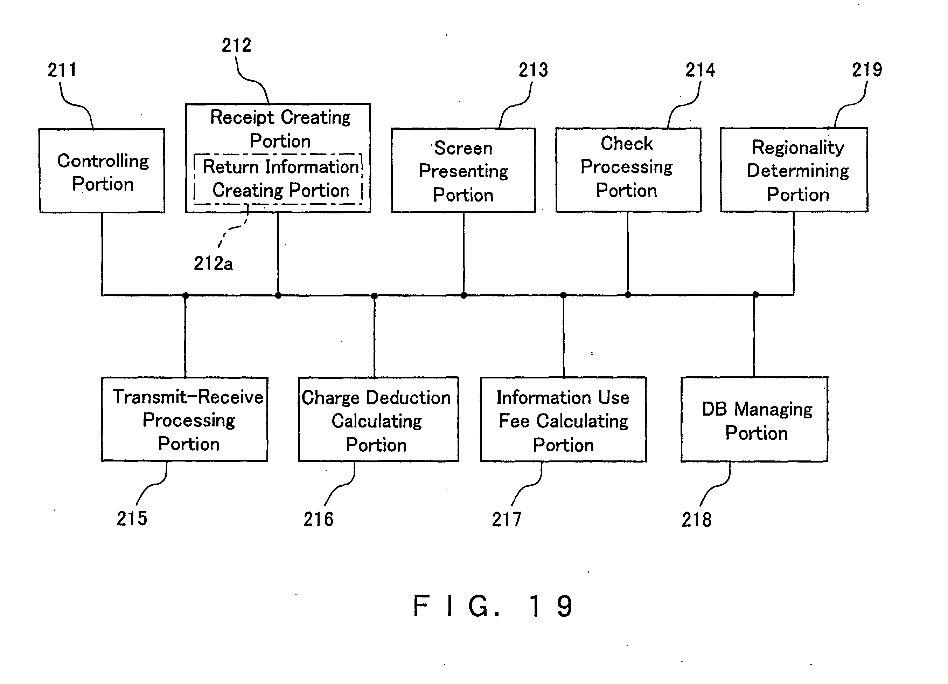

For this purpose, in the receipt creation support system of this embodiment, a regionality determining portion 219 (uneven-distribution determining portion), which classifies for every district the return information collected from the respective medical institutions into the error adjustment DB server 23, is provided to the application server 21 of the server system 2 as shown in FIG. 19.

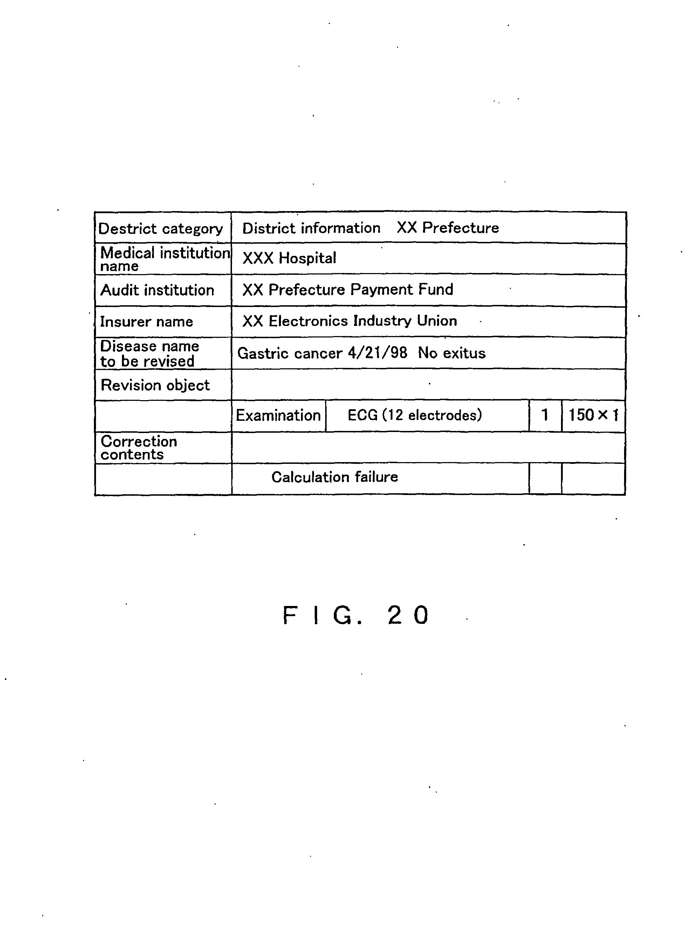

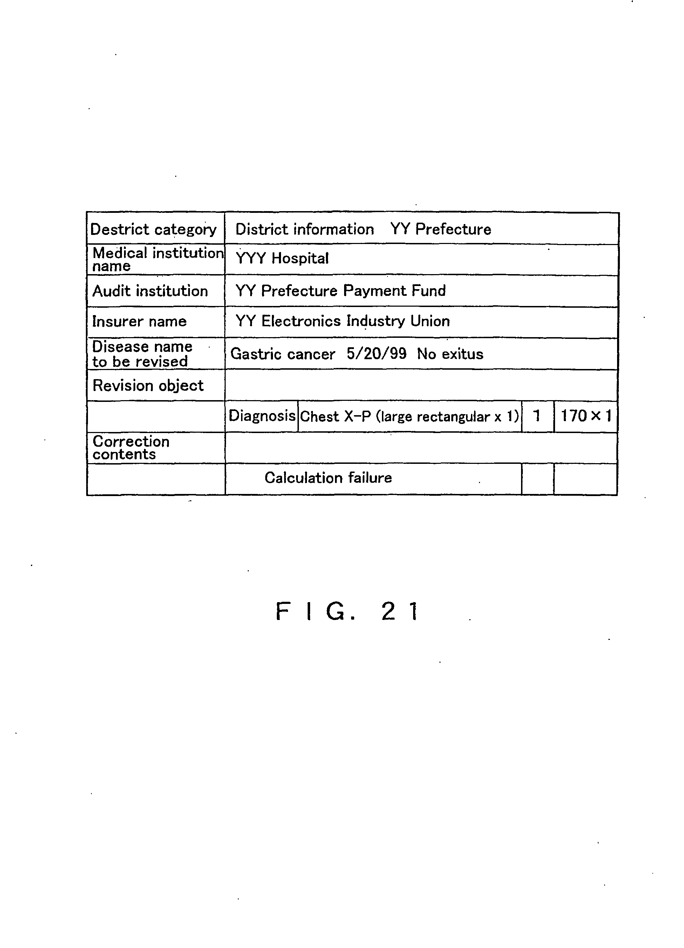

As shown in FIG. 20 and 21, in the return information registered in the error adjustment DB server 23, a district category item for representing regional uneven distribution of the return information is provided. When the return information is district information, the district to which the return information is unique is also registered in the district category item. For example, it is presented that the return information shown in FIG. 20 is unique to the XX prefecture, while the return information in FIG. 21 is information unique to the YY prefecture. The attribute value in the district category item is any of the national common information, prefecture information, and region information.

Therefore, in step S24 of FIG. 5 referred to in the first embodiment, the check processing portion 214 contrasts a district where the medical institution exists and the district category item of the return information of the error adjustment DB server 23 so as to select a piece of return information to be referred to at a time of the check.

Alternatively, it is also possible to select between limiting the return information to be referred to at the time of the check, to the district information (prefecture information and/or region information) or referring to all of the return information in the error adjustment DB server 23 without limitations. Thereby the check processing portion 214 in the application server 21 performs a check process corresponding to the selection.

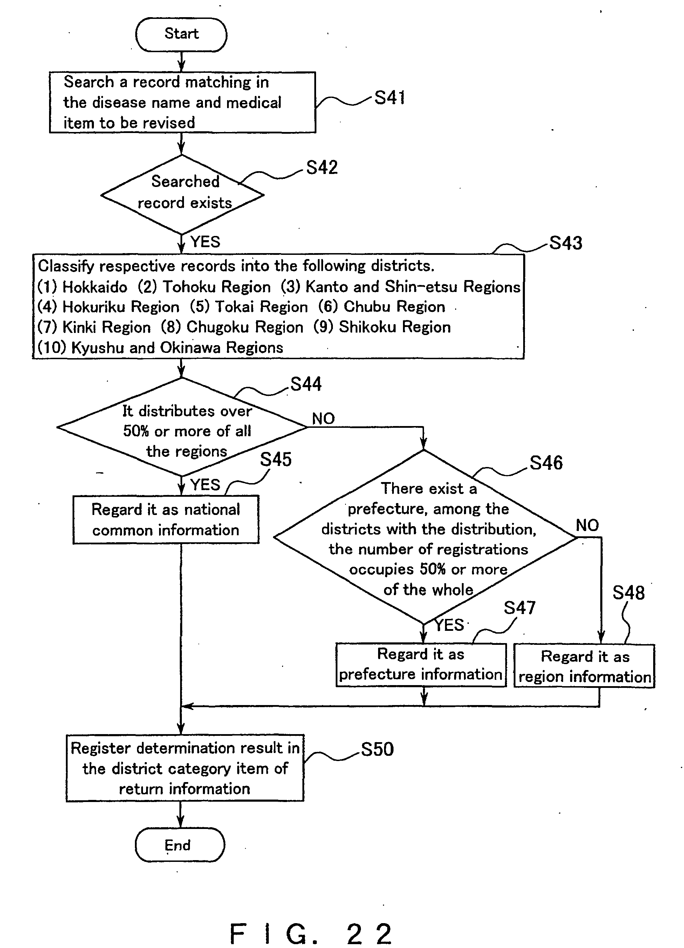

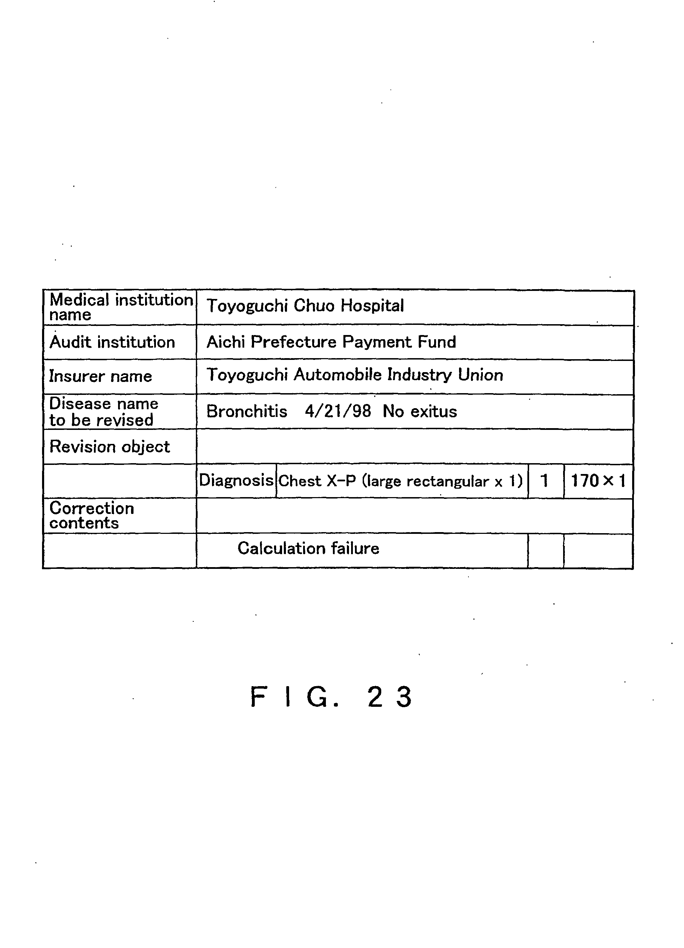

The following description with reference to FIGs. 22 and 23 concerns a procedure of a process in which the regionality determining portion 219 determines the regional uneven distribution of return information registered in the error adjustment DB server 23 and records the results in the district category item.

This process is started by the regionality determining portion 219 under the control of the controlling portion 211, when new return information is registered in the error adjustment DB server 23. FIG. 22 is a flow chart showing an operation procedure of the regionality determining portion 219. First, the regionality determining portion 219 searches the newly-registered return information and return information matching in the disease name and a medical item to be revised, among return information records (including the new return information record that is to be registered this time) registered in the error adjustment DB server 23 (step S41).

When a search record exists (YES in step S42), all the corresponding records are extracted from the error adjustment DB server 23. And, depending on the contents of the district category items of the respective records, the extracted records are classified into regions of (1) Hokkaido, (2) Tohoku Region, (3) Kanto and Shin-etsu Regions, (4) Hokuriku Region, (5) Tokai Region, (6) Chubu Region, (7), Kinki Region, (8) Chugoku Region, (9) Shikoku Region, and (10) Kyushu and Okinawa Region (step S43).

Next, the regionality determining portion 219 observes the number of the records classified into the respective regions (1)-(10), and for a case that the records are distributed in 50% or more of all the regions (that is, 5 or more of the above-identified regions (1)-(10)) (YES in step S44), this return information is determined as national common information (step S45).

On the other hand, when the determination in step S44 is NO, the regionality determining portion 219 further determines whether there exist any prefectures, among the regions (1)-(10), in which the number of the corresponding records accounts for at least 50% of the whole records (step S46). If the determination result is YES, this return information is determined as prefecture information (step S47). If the determination result in step S46 is NO, this return information is determined as region information (step S48).

As described above, when the determination about the regional uneven distribution is completed, the regionality determining portion 219 registers the determination result in the district category item of each return information (step S49), and completes the process. As a result, the attribute value of the district category item of the return information will be "national common information", "prefecture information (and the corresponding prefectures)", and "region information (and the corresponding regions)". For the cases of the prefecture information or the region information, it is also possible to calculate, as a relative frequency for prefectures or for regions, a rate that the number of the records classified into either the prefecture information or the region information with respect to the whole records of return information, and to register the value also in the district category item.

A specific example about how the regional uneven distribution of the return information is determined will be described below in accordance with the flow chart in FIG. 22. Here, the return information having contents as shown in FIG. 23 is registered from the client system 1 of a hospital located in Aichi prefecture into the error adjustment DB server 23. This return information is a hypothetical example and not based on the actual audit standards in Aichi prefecture.

In step S41, it is assumed that in the return information records of the error adjustment DB server 23, there are 45 subjects of return information that corresponds to the above-identified return information in the disease name (bronchitis) and the medical item to be revised (diagnosis: chest X-P (large rectangular x 1)).

Furthermore, as a result of the classification by step S43, the distribution of the records are assumed as follows.

| | |
|---|---|
| Hokkaido | 0 |
| Tohoku | 0 |
| Kanto and Shin-etsu | 10 |
| Hokuriku | 3 |
| Chubu | 0 |
| Tokai | 9 |
| Kinki | 23 |
| Chugoku | 0 |
| Shikoku | 0 |
| Kyushu and Okinawa | 0 |

In this case, since the records are distributed in only four regions among all of the 10 regions, the determination result in step S44 becomes NO.

Furthermore, it is assumed that the distribution in prefectures of the four regions is as follows.
Kanto and Shin-etsu: 4 in Tokyo, 4 in Kanagawa, and 2 in Chiba
Hokuriku: 2 in Ishikawa, and 1 in Fukui
Tokai: 4 in Aichi, 4 in Shizuoka, and 1 in Gifu
Kinki: 10 in Osaka, 8 in Kyoto, and 5 in Hyogo

In this case, since there are no prefectures accounting for at least 50% in each region, the determination result in step S46 becomes NO. That is, all of the return informations are determined as region informations (step S48).

As a result, for example, for all of the ten subjects of the return information records distributed in the Kanto and Shin-etsu regions, the attributes of the district category items become region informations, and the 'Kanto and Shin-etsu' is registered as the region name.

As described above, according to the configuration of the embodiment, the regionality determining portion 219 extracts the identical return information from the return information registered in the error adjustment DB server 23. By checking the district distribution of these return informations, it determines whether the return information is national common information, region information or prefecture information, and registers the result in the error adjustment DB server 23.

Accordingly, the accuracy in the receipt check process can be improved by extracting, from the error adjustment DB server 23, only return information corresponding to the district where the medical institution exists, and performing a check of the receipt description data on the basis of the extracted return information, at the time of the receipt check process.

In the configuration according to the embodiment, in the charge reduction calculating portion 216 and the information use fee calculating portion 218 of the application server 21, the basic fee or the like per subject of return information can be varied at the time of calculation of the reduction or the information use fee, depending on which of the national information, region information or the prefecture information each return information corresponds to.

For example, in a case that the charge reduction calculating portion 216 decides the reduction in accordance with the number of subjects of the return information provided by medical institutions, it can be considered the reduction per return information is set to be higher for region information than for national information, and higher for prefecture information than for the region information. Similarly, when the information use fee calculating portion 218 calculates the information use fee corresponding to the number of uses of the return information, it can be considered the use fee per subject is set to be higher for region information than for national information, and higher for prefecture information than for the region information. This is based on an idea that from a viewpoint of the specialty (characteristic) of the return information, the prefecture information is regarded as the most useful information for the medical institutions as users.

In the above examples of the first and second embodiments, all of the receipt creating portion, the return information creating portion, the screen presenting portion and the check processing portion are arranged in the application server 21 of the server system 2. Alternatively, these portions can be arranged in a medical institution (client system).

That is, the return information storing portion (error adjustment DB server 23) must be arranged in the server system 2 as it should be used commonly by all of the medical institutions. On the other hand, regarding each of the receipt creating portion, the return information creating portion, the screen presenting portion and the check processing portion, they can demonstrate their own functions when being arranged in either the client system of each medical institution or the server system 2.

Although a medical institution is shown as a receipt creation institution in the above embodiments, the receipt creation institution will not be limited to a medical institution, but it can be an ethical pharmacy or the like.

### (Third Embodiment)

Another embodiment of the present invention will be explained below by referring to the attached drawings. An input support system in this embodiment denotes a managerial decision request system used for creating a managerial decision request within a firm.

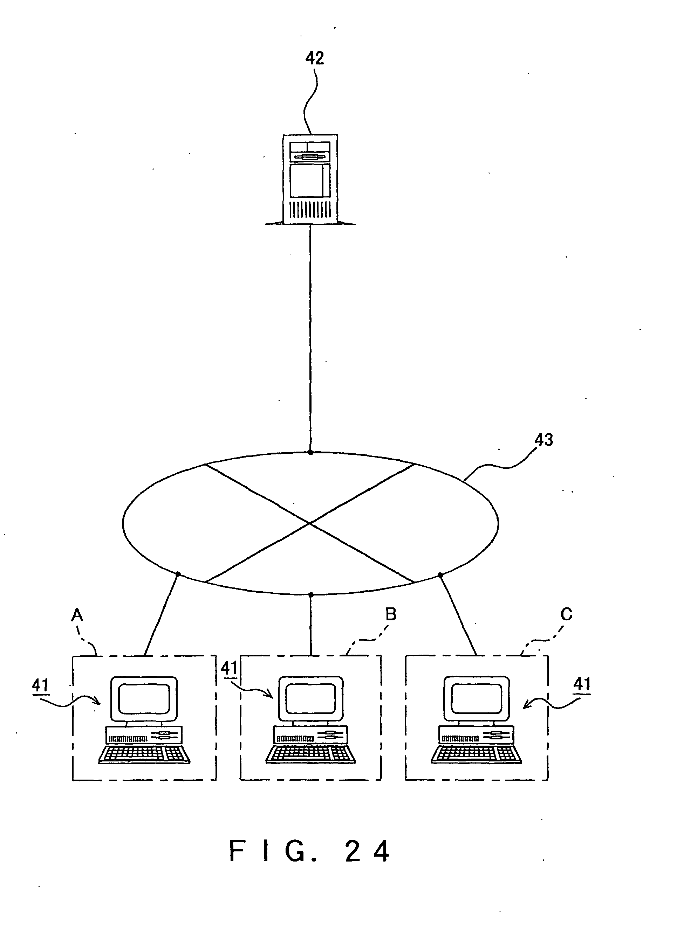

As shown in FIG. 24, the managerial decision request system of this embodiment has a configuration that computers 41 provided in respective sections A, B, C,... of the firm and a server 42 are connected through an in-house LAN 43.

In FIG. 24, for a configuration example for a client, only computers 41 as so-called desktop personal computers having displays and keyboards are shown, while peripheral equipment such as printers is omitted in the figure. The type and the scale of the client are not limited to the example in FIG. 24, but any suitable arbitrary computer system can be applied. Though the client and the server are connected through an in-house LAN in this configuration, e.g., for a case that the respective sections are positioned remotely, the connection can be provided through WAN (Wide Area Network) such as the Internet. Furthermore, while this embodiment shows an example in which the server system is configured by one server 42, the server system can be configured with plural servers.

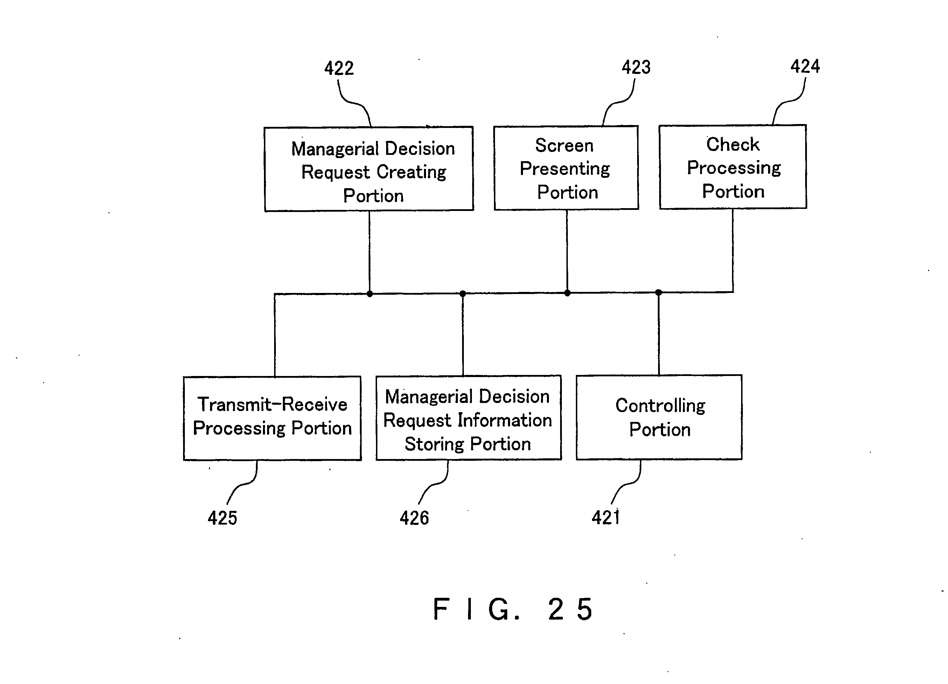

As shown in FIG. 25, the server 42 includes a controlling portion 421, a managerial decision request creating portion 422, a screen presenting portion 423, a check processing portion 424, a transmit-receive processing portion 425, and a managerial decision request information storing portion 426.

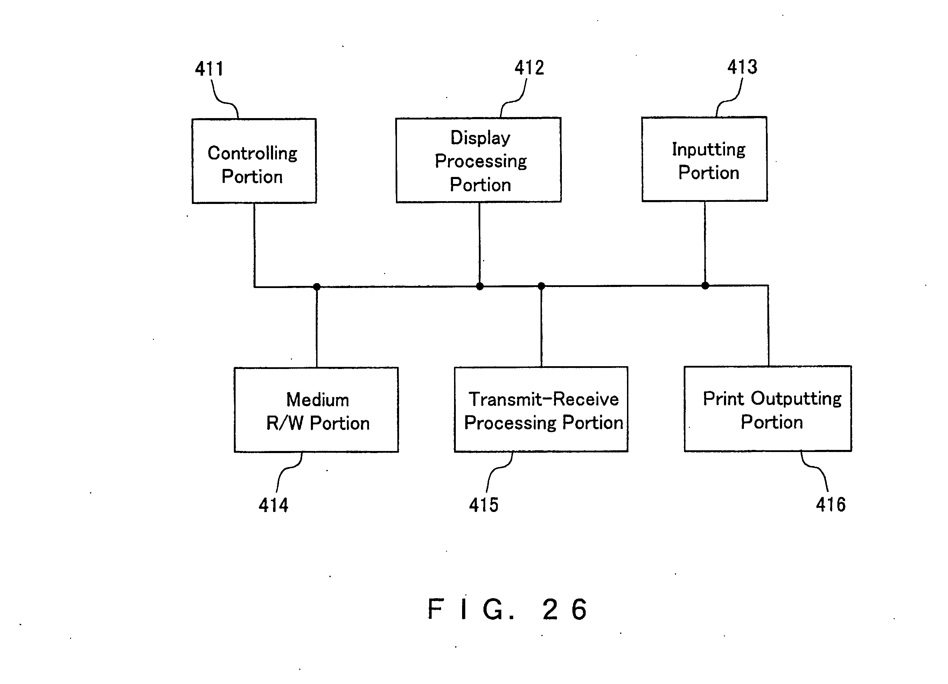

As shown in FIG. 26, each computer 41 includes a controlling portion 411, a display processing portion 412, an inputting portion 413, a medium R/W portion 414, a transmit-receive processing portion 415, and a print outputting portion 416.

The following explanation is about operations of the managerial decision request system.

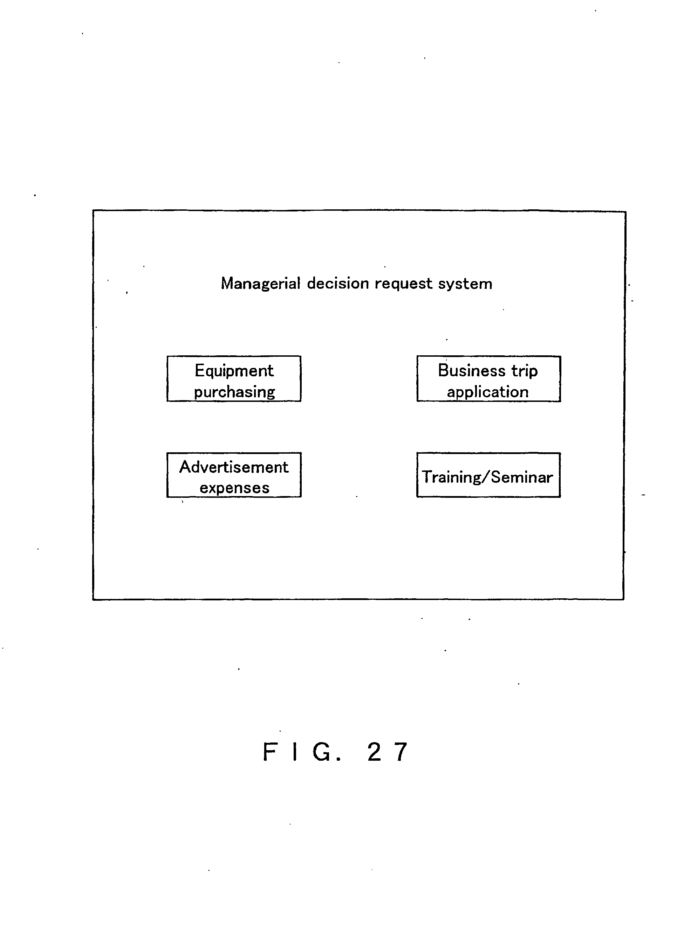

An operator who wants to create a managerial decision request performs an operation for calling from a computer 41 an initial screen of a managerial decision request system as shown in FIG. 27. This initial screen is sent from the screen presenting portion 423 of the server 42 to the transmit-receive processing portion 425 and also to the computer 43 through the LAN 43, received by the transmit-receive processing portion 415 and indicated on a display of the computer 41 by means of the display processing portion 412.

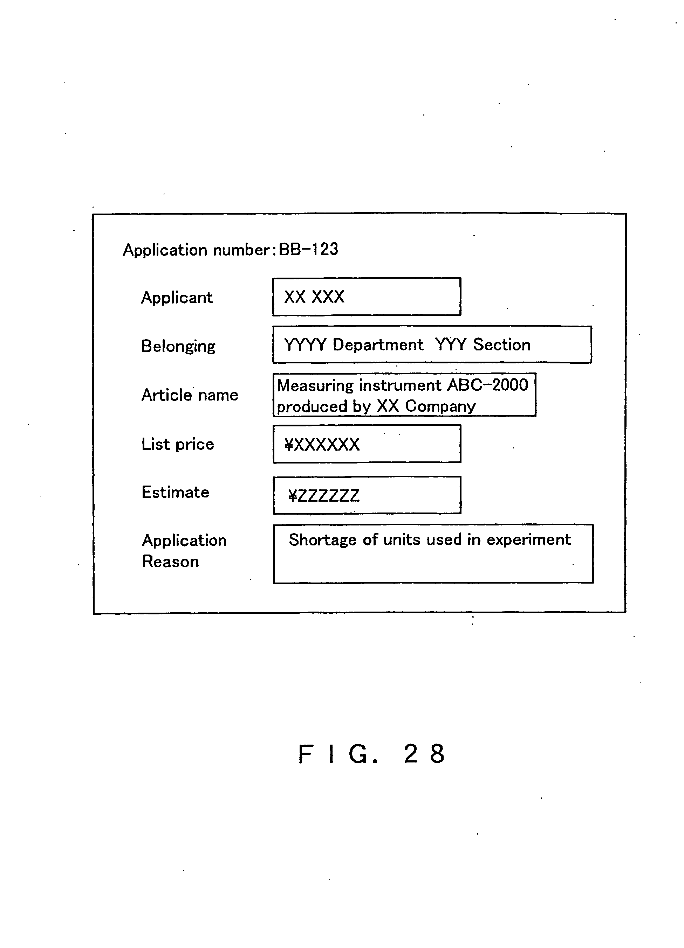

On the initial screen of the managerial decision request system, as shown in FIG. 27, managerial decision request items such as equipment purchasing expenses, advertisement expenses, business trip expenses, training/seminar expenses are displayed. The operator clicks an item required to apply for a managerial decision request. For example, for creating a managerial decision request for equipment-purchasing expenses, the operator clicks the part of "Equipment purchasing' so that an input screen as shown in FIG. 28 is provided from the screen presenting portion 423 of the server 42 to the computer 41. Then, the operator fills in the respective columns on this input screen by using a keyboard or the like of the computer 41.

The data inputted into the respective columns are sent from the computer 41 to the server 42, and checked at the check processing portion 424. The check processing portion 424 compares the inputted data and the managerial decision request information stored in the managerial decision request information storing portion 426.

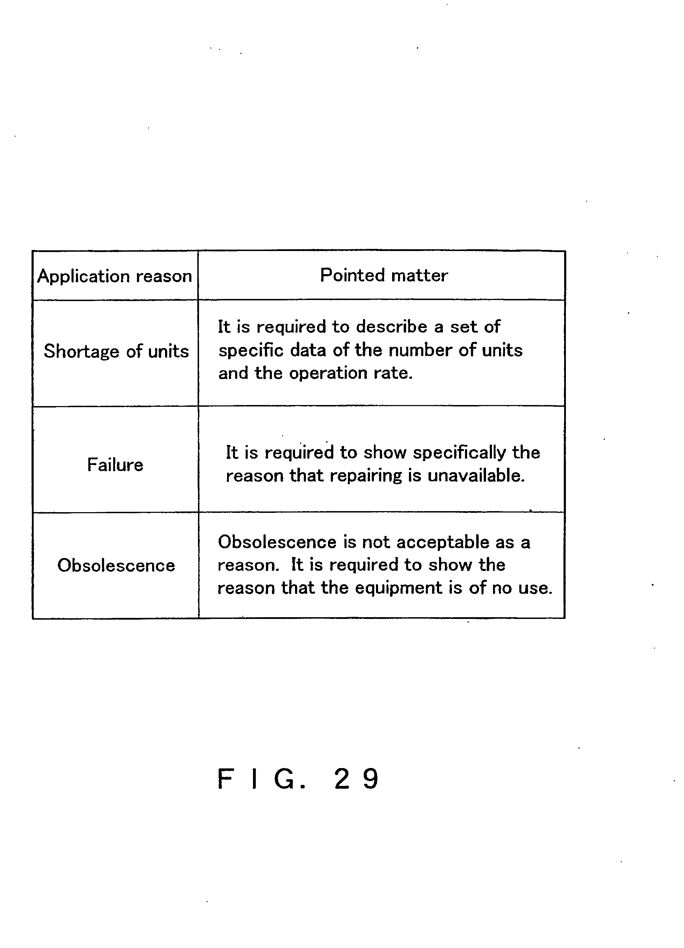

The managerial decision request information storing portion 426 is a database of accumulated matters (managerial decision request information) identified as a return reason at the time that a submitted managerial decision request was remanded due to the defects in description. Here, FIG. 29 shows an example of managerial decision request information stored in the managerial decision request information storing portion 426. The managerial decision request information shown in FIG. 29 relates to a managerial decision request for equipment purchasing expenses. This managerial decision request information is sent from the computer 41 of every section to the server 42 at the time of remand of the managerial decision request, accumulated and stored in the managerial decision request information storing portion 426.

The check processing portion 424 analyzes data inputted in the application reason column of the input screen as shown in FIG. 28. For example, in the case of the input data shown in FIG. 28, a keyword is extracted from a sentence of "Shortage of units used in experiment" and analyzed to determine that the application reason is "Shortage of units". Then, the managerial decision request information of the managerial decision request information storing portion 426 is referred to for searching managerial decision request information having an application reason of "Shortage of units". Here, as shown in FIG. 29, there is managerial decision request information with an application reason of "Shortage of units", and the check processing portion 424 passes the identified matters of the managerial decision request information to the managerial decision request creating portion 422.

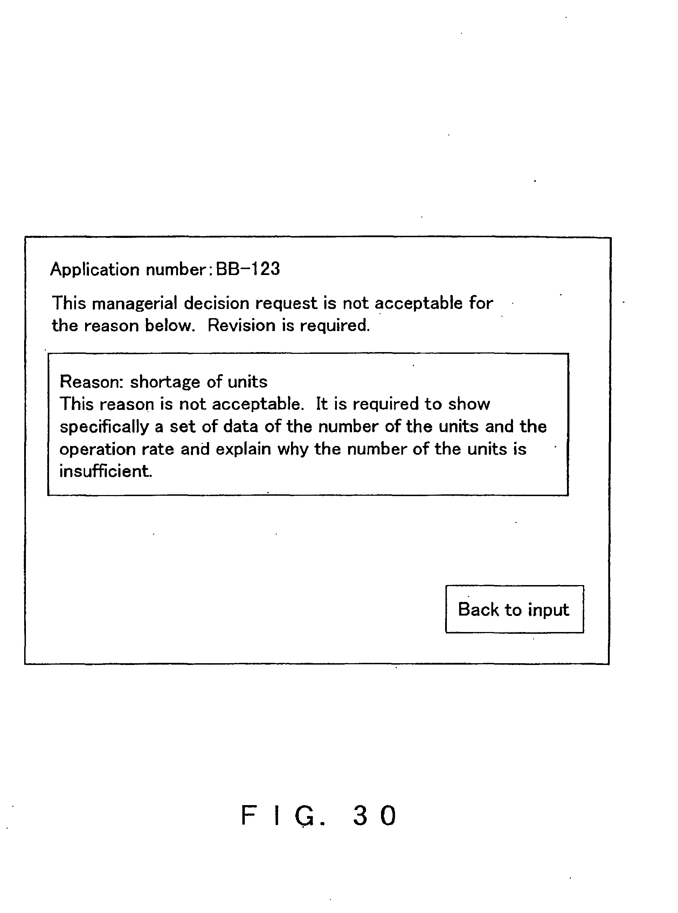

Based on the information accepted from the check processing portion 424, the managerial decision request creating portion 422 creates a revision direction screen as shown in FIG. 30, and passes it to the screen presenting portion 423. This screen is sent from the screen presenting portion 423 to the computers 41 and indicated on the display.

The operator confirms this screen with the displays of the computer 41 and clicks the part of "Back to input", so that the input screen as shown in FIG. 28 is displayed again. Then, he revises the description in the column of the application reason of the input screen. At this time, it is preferable to configure for multi-window displaying the revision direction screen of FIG. 30 and the input screen of FIG. 28, so the operator can perform revision input while observing the revision direction.

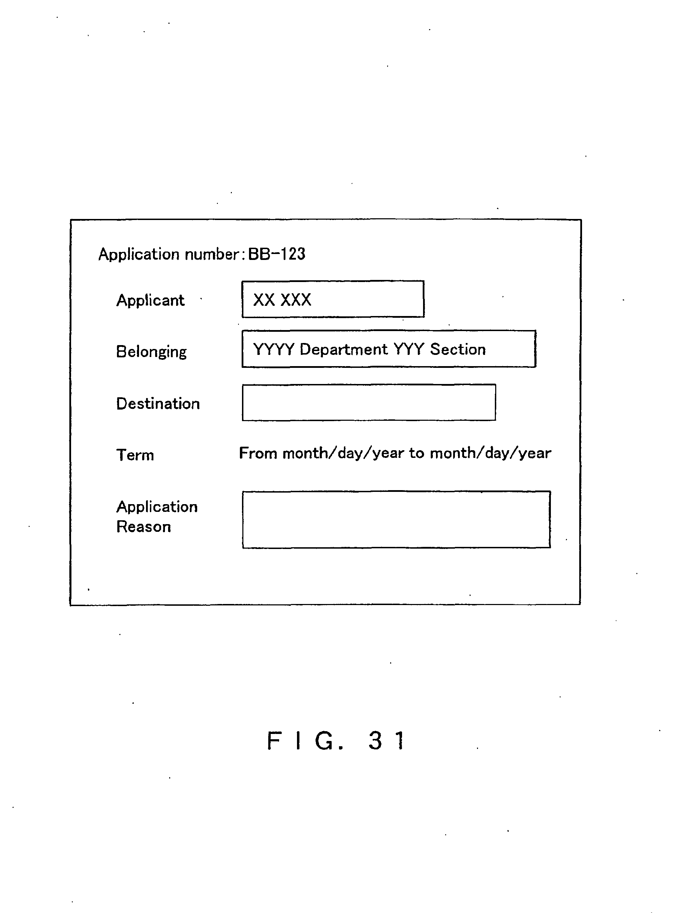

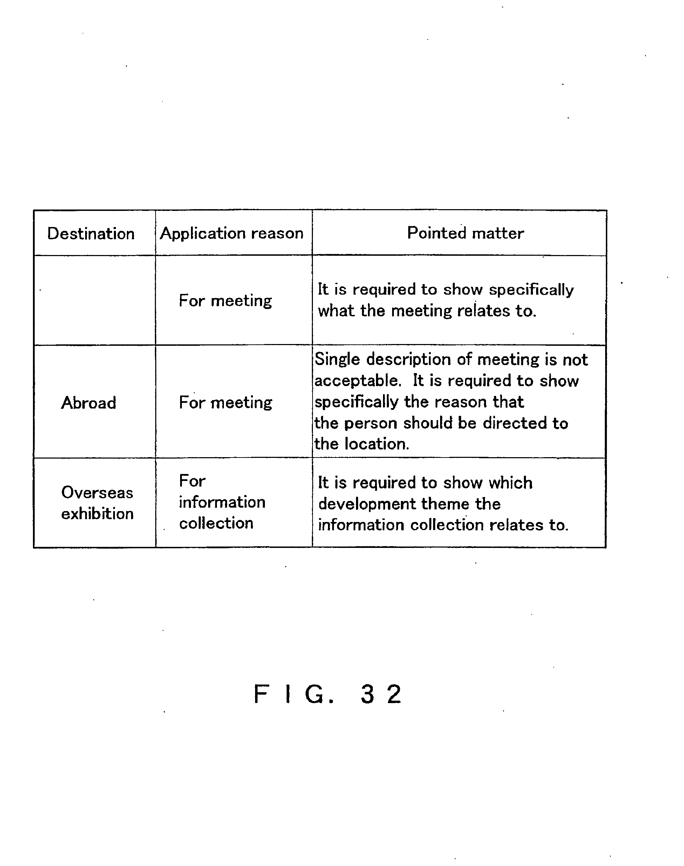

Furthermore, in the initial screen as shown in FIG. 27, an input screen as shown in FIG. 31 is displayed when the operator selects an application of business trip. FIG. 32 shows an example of managerial decision request information stored in the managerial decision request information storing portion 426 regarding the business trip application. For example, on the input screen shown in FIG. 31, when the operator inputs "Abroad" in the destination column, and simply "For meeting" in the application reason column, the check processing portion 426 refers to the managerial decision request information as shown in FIG. 32, and the managerial decision request creating portion 422 creates a revision direction screen including a message that "Single description of meeting is not acceptable. It is required to show specifically the reason that the person should be directed to the location.", and allows the computers 41 to display the message. Accordingly, the operator recognizes to go back to the input screen and revise the description of the application reason column to, for example, "For meeting on issue of quality of a product XX and confirmation of quality of the production line".

As described above, according to the managerial decision request system in the embodiment, it is possible to detect defects in input data and perform a suitable revision before submitting a managerial decision request, which is realized by accumulating as managerial decision request, in the managerial decision request information storing portion 426, the reason that past managerial decision requests have been remanded, and by comparing input data with this managerial decision request information. This can serve to save some work for remand of a managerial decision request caused by defects and resubmission, thereby providing a managerial decision request system to improve efficiency of in-house business.

### Industrial Applicability

As described above, the present invention can provide, by checking input data on the basis of defective data information based on past examples, an input support system enabling to suitably revise defects of the input data.

## Claims

1. An input support system comprising:
a client system for allowing data input work, and
a server system connected to at least one client system, which supports the data input work at the client system by using defective data information collected from each client system;
wherein a defective data information storing portion for storing the defective data information as a record comprising, as items, data to be revised and revised data is provided in at least one of the server system and the client system, and
a check processing portion for performing a process of checking the input data is provided in either the client system or the server system, the check process denotes a process of contrasting the defective data information at the defective data information storing portion and input data at the client system, and when there are any input data the same as data included in the item of data to be revised in the defective data information, performing a defect prevention process or a defect revision process with respect to the input data.

2. The input support system according to claim 1, wherein the defect prevention process by the check processing portion comprises a process of extracting defective data information relating to the data to be processed from the defective data information storing portion and displaying the same.

3. The input support system according to claim 1 or 2, wherein the client system comprises a defective data information transmitting portion for transmitting the defective data information relating to the input data of the client system to the server system, and
the server system stores remand information transmitted from the client system, in the defective data information storing portion.

4. An input support system comprising:
a client system provided in a receipt creation institution that performs receipt creation business, and
a server system that is connected to a client system of at least one receipt creation institution through a communications line and supports receipt creation business in the client system by using receipt return information collected from each receipt creation institution;
wherein a return information storing portion for storing the return information as a record including, as items, data to be returned and revised data is provided in the server system, and
a check processing portion for checking description data of a receipt before submission is provided in either the client system or the server system, the check process denotes a process of contracting the return information of the return information storing portion and the description data in the receipt before submission, and when there are description data that are the same as data included in the item of the data to be returned in the return information, performing a defect prevention process or a defect revision process with respect to the description data as data to be processed.

5. The input support system according to claim 4, wherein the defect prevention process by the check processing portion comprises a process of extracting return information relating to the data to be processed from the return information storing portion and displaying the same.

6. The input support system according to claim 4, wherein the defect revision process by the check processing portion comprises a process of replacing the data same as the data to be processed, by revised data of return information included in the item of data to be revised.

7. The input support system according to any of claims 4 to 6, wherein the client system comprises a return information transmitting portion for transmitting return information with respect to a receipt of the receipt creation institution to the server system through the communications line, and
the server system comprises a return information transmitting portion for transmitting return information with respect to the receipt of the receipt creation institution, to the server system through the communications line.

8. The input support system according to any of claims 4 to 7, wherein the record of each return information in the return information storing portion further comprises an item representing regional uneven distribution of the return information, and
the check processing portion selects return information to be referred to at the time of the check process by contrasting the district where the receipt creation institution creating the receipt exists and an item of regional uneven distribution for each return information.

9. The input support system according to claim 8, wherein the server system further comprises an uneven-distribution determining portion for determining the regional uneven distribution of the return information having identical contents in the return information storing portion and registering the determination result in the item of the regionality of the return information.

10. The input support system according to claim 8 or 9, wherein a regional unit of the uneven distribution is administered by an audit institution.

11. The input support system according to any of claims 8 to 10, wherein the attribute of the item for the uneven distribution of the return information record is selected from local information, region information and national common information, corresponding to the uneven distribution of the return information.

12. The input support system according to any of claims 4 to 11, wherein the client system comprises a selecting portion for transmitting to the server system a use flag representing whether to use at the receipt creation institution each return information stored in the return information storing portion;
the record of each return information in the return information storing portion further includes a use flag item representing a use flag transmitted from the selecting portion of each receipt creation institution; and
the check processing portion refers to the use flag item and selects return information to be referred to at the time of the check process.

13. The input support system according to any of claims 4 to 12, further comprising an input screen presenting portion for presenting an input screen that supports input of description data of a receipt, and a creating portion for creating a receipt based on the data inputted into the input screen.

14. The input support system according to claim 13, wherein the input screen presenting portion and the creating portion are provided in the client system.

15. The input support system according to claim 13, wherein the input screen presenting portion is provided in the client system and the creating portion is provided in the server system, and
description data of the receipt inputted into the input screen is transmitted through the communications line to the creating portion of the server system.

16. The input support system according to claim 13, wherein
the input screen presenting portion is provided in the server system and the creating portion is provided in the client system;
the input screen presenting portion transmits input screen data described in a page-description language to the client system through the communications line;
the client system comprises a display processing portion for displaying the input screen by analyzing the input screen data, and the display processing portion sends to the creating portion the description data of the receipt inputted into the input screen.

17. The input support system according to claim 13, wherein the input screen presenting portion and the creating portion are provided in the server system;
the input screen presenting portion transmits the input screen data described in a page-description language to the client system through the communications line;
the client system comprises a display processing portion for displaying an input screen by analyzing the input screen data, and the display processing portion sends description data of the receipt inputted into the input screen to the creating portion of the server system through the communications line.

18. The input support system according to any of claims 13 to 17, wherein
the input screen presenting portion acquires description data of a submitted receipt and displays in the input screen;
the creating portion creates a receipt for resubmission on the basis of description data revised on the input screen; and
the creating portion comprises a return information creating portion for creating a new return information record to be registered in the return information storing portion, regarding the description data to be revised as the item of data to be returned and also regarding the revised data as an item of revised data.

19. The input support system according to any of claims 13 to 18, wherein every time the description data of the receipt are inputted into the input screen, the check processing portion performs the check process with respect to the description data.

20. The input support system according to any of claims 4 to 19, wherein
the client system or the server system comprises further a reading portion for reading the created receipt from a storage medium in which the receipt is stored;
the check processing portion performs the check process in a batch with respect to whole description data of at least one receipt that are read out from the storage medium by the reading portion.

21. The input support system according to claim 20, wherein a result of the check process performed in a batch by the check processing portion is compiled in a list and presented to each receipt creation institution.

22. The input support system according to any of claims 4 to 21, wherein the server system comprises a charge reduction calculating portion for deciding a reduction of a use fee to be imposed on every receipt creation institution, corresponding to at least one of the number of subjects and the frequency that the return information is presented from every client system to the server system.

23. The input support system according to any of claims 4 to 23, wherein the server system comprises a return information use fee calculating portion for deciding return information use fee to be imposed on every receipt creation institution, corresponding to the frequency of use of the return information of the return information storing portion in every client system.
